(19) Europäisches Patentamt
European Patent Office
Office européen des brevets

(11) EP 1 616 558 A1

(12) DEMANDE DE BREVET EUROPEEN

(43) Date de publication:
**18.01.2006 Bulletin 2006/03**

(51) Int Cl.:
***A61K 8/81*** *(2006.01)* ***A61K 8/898*** *(2006.01)*
***A61Q 1/06*** *(2006.01)*

(21) Numéro de dépôt: **05291151.8**

(22) Date de dépôt: **27.05.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **16.07.2004 FR 0451551**

(71) Demandeur: **L'OREAL**
**75008 Paris (FR)**

(72) Inventeur: **Blin, Xavier**
**75015 Paris (FR)**

(74) Mandataire: **Chantraine, Sylvie Hélène**
**L'OREAL - D.I.P.I.**
**25-29, Quai Aulagnier**
**92600 Asnieres (FR)**

(54) **Produit cosmétique bicouche comprenant un polymère de silicone**

(57) L'invention se rapporte à un produit cosmétique contenant une première et une seconde compositions, la première composition comprenant un polymère de silicone particulier et la seconde composition comprenant un milieu cosmétiquement acceptable.

L'invention se rapporte également à un procédé de maquillage ainsi qu'à un kit de maquillage contenant ledit produit. Ce dernier est en particulier un rouge à lèvres, un mascara ou un vernis à ongles.

EP 1 616 558 A1

## Description

La présente invention se rapporte à un produit cosmétique comprenant au moins deux compositions qui peuvent être appliquées successivement sur la peau aussi bien du visage que du corps, sur les paupières inférieures et supérieures, sur les lèvres et sur les phanères comme les ongles, les sourcils, les cils ou les cheveux. La présente invention concerne également un procédé de maquillage du visage et du corps mettant en oeuvre ces deux compositions.

Chaque composition peut être un fond de teint, un fard à joues ou à paupières, un produit anti-cerne, un blush, une poudre libre ou compactée, un rouge à lèvres, un baume à lèvres, un brillant à lèvres, un crayon à lèvres ou à yeux, un mascara, un eye-liner, un vernis à ongles ou encore un produit de maquillage du corps ou de coloration de la peau.

Un objectif de la présente invention est de proposer une composition qui soit à la fois de bonne tenue et brillante.

Une bonne tenue se traduit notamment par a) une bonne tenue de la couleur, notamment lorsque la composition entre en contact avec l'eau, la salive, la sueur, le sébum ou des corps gras, b) l'absence de transfert de la composition sur des objets avec lesquels elle entre en contact, c) l'absence de migration hors du tracé initial du maquillage, d) la tenue de la brillance au cours du temps.

Une mauvaise tenue peut se caractériser par une modification de la couleur (virage, palissement) généralement par suite d'une interaction avec le sébum et/ou la sueur sécrétés par la peau, dans le cas de fond de teint et de fard à joue, ou d'une interaction avec la salive dans le cas des rouges à lèvres. Ceci oblige l'utilisateur à se remaquiller très souvent, ce qui peut constituer une perte de temps.

Les compositions de maquillage pour les lèvres et la peau qui transfèrent peu ou pas du tout sont des compositions qui présentent l'avantage de former un dépôt qui ne se dépose pas, au moins en partie, sur les supports avec lesquels elles sont mises en contact (verre, vêtements, cigarette, tissus).

Les compositions sans transfert connues sont généralement à base de résines de silicone et d'huiles de silicone volatiles et, bien que présentant des propriétés de tenue améliorées, ont l'inconvénient de laisser sur la peau et les lèvres, après évaporation des huiles de silicone volatiles, un film qui devient inconfortable au cours du temps (sensation de dessèchement et de tiraillement), écartant un certain nombre de femmes de ce type de rouge à lèvres.

En outre, ces compositions à base d'huiles volatiles de silicone et de résines siliconées conduisent à des films colorés mats. Or, les femmes sont aujourd'hui à la recherche de produits, notamment de coloration des lèvres ou des paupières, brillants, tout en étant de bonne tenue, et sans transfert.

On peut aussi citer la demande de brevet WO-A-97/17057 qui décrit une méthode pour augmenter les propriétés de tenue et de non transfert consistant à appliquer deux compositions l'une sur l'autre. La composition à appliquer possède un paramètre de solubilité global d'Hildebrand inférieur à 8,5 $(cal/cm^3)^{1/2}$, et la composition à appliquer à titre de surcouche doit contenir des huiles dont le coefficient de partage calculé ClogP est au moins égal à 13.

Le brevet US-A-6 001 374 propose un système de maquillage multicouche consistant à utiliser une composition contenant une résine soluble dans l'alcool et insoluble dans l'eau, que l'on peut appliquer comme couche de base ou en surcouche, et présentant l'avantage de ne pas tâcher un objet mis au contact du maquillage et de résister à l'eau et aux frottements, tout en ayant un certain brillant. Cependant, cette composition contient un alcool hydrosoluble, en particulier l'éthanol, composé qui présente un caractère irritant, desséchant pour la peau et plus spécialement pour les lèvres, et qui est particulièrement inconfortable lorsque la peau ou les lèvres sont abîmées.

Dans la demande WO 02/067877, il est décrit une méthode pour améliorer les propriétés esthétiques d'une composition non transfert consistant à appliquer une deuxième composition sur un film de composition non transfert. La deuxième composition ne doit pas interagir chimiquement avec la composition non transfert afin de ne pas altérer ses propriétés cosmétiques. Certains produits décrits dans ce document ont une odeur désagréable et sont collants. D'autres produits ne sont pas assez brillants.

La présente invention a pour but de proposer une nouvelle voie de formulation d'un produit cosmétique, en particulier un produit de maquillage, comportant deux compositions à appliquer successivement l'une sur l'autre. Ce produit cosmétique peut avantageusement présenter de bonnes propriétés de brillance, de tenue et/ou de confort.

La présente invention a encore pour but de proposer un produit cosmétique, en particulier un produit de maquillage, qui réunisse avantageusement à la fois les propriétés de tenue de la couleur, tenue de la brillance dans le temps, non transfert, non migration, confort, et brillance.

Le demandeur a constaté que c es objets pouvaient être atteints e n associant u ne première composition comprenant un polymère siliconé particulier, et une seconde composition comprenant un milieu cosmétiquement acceptable. La seconde composition contient en particulier au moins une huile.

Ainsi, selon un des aspects de l'invention, les compositions de l'invention permettent d'obtenir un rendu cosmétique très brillant à l'application et dans le temps, qui ne migre pas, ne transfère pas, de bonne tenue, tout en étant confortable à l'application et dans le temps.

Les propriétés de tenue de la couleur, de non transfert et de non migration, alliées à l'aspect brillant, font de la composition selon l'invention un produit particulièrement approprié pour réaliser des produits de maquillage des lèvres tels que rouges à lèvres, brillants à lèvres, des produits de maquillage des yeux tels que mascara, eye-liners, fards à

paupières, et des produits de maquillage des ongles ou des cheveux.

Un objet de la présente invention est donc un produit cosmétique destiné à être appliqué sur la peau, notamment la peau du visage, du cou, les lèvres, les paupières, comprenant une première et une seconde compositions, la première composition contenant au moins un polymère de silicone de formule générale (I) :

$$R^1_a\, R^2_b\, R^3_c\, SiO_{(4-a-b-c)/2} \qquad (I)$$

dans laquelle :

- a, b et c sont tels que a varie de 1 à 2,5 ; b et c, indépendamment l'un de l'autre, varient de 0,001 à 1,5 ;
- $R^1$, identique ou différent, est choisi parmi :
  - les radicaux alkyle en $C_1$ à $C_{30}$, le cas échéant substitués par un ou plusieurs atomes de fluor, groupements amino et/ou carboxy,
  - les radicaux aryle, aralkyle, et
  - les radicaux de formule générale (II) :

    $$-C_dH_{2d}-O-(C_2H_4O)_e(C_3H_6O)_fR^4 \qquad (II)$$

    avec :

    - $R^4$ étant un radical hydrocarboné en $C_1$ à $C_{30}$ ou un radical $R^5$-(CO)- avec $R^5$ étant un radical hydrocarboné en $C_1$ à $C_{30}$, et
    - d, e et f étant des entiers tels que d varie de 0 à 15, e et f, indépendamment l'un de l'autre, varient de 0 à 50,
  - et leurs combinaisons,
- $R^2$ est un radical choisi parmi les radicaux représenté par la formule générale (III) :

  $$-Q-O-X \qquad (III)$$

  avec :

  - Q étant un radical hydrocarboné bivalent en $C_2$ à $C_{20}$ pouvant inclure au moins une liaison éther et/ou au moins une liaison ester, et
  - X étant un radical hydrocarboné polyhydroxylé,
- $R^3$ est radical choisi parmi
  - les radicaux alkyle en $C_1$ à $C_{30}$, le cas échéant substitués par un ou plusieurs atomes de fluor, groupements amino et/ou carboxy,
  - les radicaux aryle, aralkyle,
  - les groupements organosiloxane de formule générale (IV):

    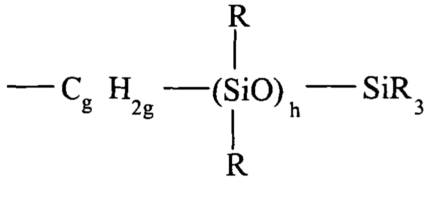

    (IV)

    avec :

    - chacun des radicaux R représentant un radical choisi parmi les radicaux alkyle en $C_1$ à $C_{30}$, le cas échéant substitués par un ou plusieurs a tomes d e fluor, e t les radicaux aryle et aralkyle,
    - g et h étant des entiers tels que g varie de 1 à 5 et h varie de 0 à 500,

et la seconde composition distincte de la première comprenant un milieu cosmétiquement acceptable.

Un autre objet de la présente invention est un produit cosmétique destiné à être appliqué sur les phanères, notamment les ongles, les cheveux ou les sourcils, comprenant une première et une seconde compositions, la première composition contenant, dans un milieu liquide organique cosmétiquement acceptable, au moins un polymère de silicone tel que décrit précédemment, et la seconde composition distincte de la première comprenant un milieu cosmétiquement acceptable.

Le produit de l'invention est en particulier un produit de maquillage de la peau, des ongles ou des cheveux.

Par produit de maquillage, on entend un produit contenant un agent colorant permettant le dépôt d'une couleur sur une matière kératinique (la peau ou les phanères) de personne humaine par l'application sur la matière kératinique de produits tels que des rouges à lèvres, des fards, des eye-liners, des fonds de teint ou des autobronzants, des produits de maquillage semi permanent (tatouage).

Le produit selon l'invention comprend au moins deux compositions cosmétiquement acceptables conditionnées séparément ou ensemble dans un même article de conditionnement ou dans deux articles de conditionnement séparés. De préférence, ces compositions sont conditionnées séparément et avantageusement, dans des articles de conditionnement séparés.

L'objet de la présente invention est donc en particulier un produit cosmétique de maquillage se présentant sous la forme d'un rouge à lèvres, d'un fond de teint, d'un mascara, d'un fard à joues ou à paupières, d'un rouge à lèvres, d'un vernis à ongle, d'un produit ayant notamment des propriétés de soin, d'un mascara, d'un eye-liner, d'un produit anti-cernes, ou d'un produit de maquillage du corps (du type tatouage) ou des cheveux.

L'invention a aussi pour objet un kit de maquillage contenant un produit cosmétique de maquillage tel que défini précédemment, dans lequel les différentes compositions sont conditionnées séparément et sont avantageusement accompagnées de moyens d'application appropriés. Ces moyens peuvent être des pinceaux, des brosses, des stylos, des crayons, des feutres, des plumes, des éponges, des tubes et /ou des embouts mousse.

La première composition du produit selon l'invention peut constituer une couche de base appliquée sur la matière kératinique et la seconde composition une couche de dessus. Il est toutefois possible d'appliquer sous la première couche une sous couche ayant la constitution ou non de la seconde couche.
Il est aussi possible de déposer une surcouche sur la seconde couche ayant une constitution identique ou non à celle de la première couche. De préférence, le maquillage obtenu est un maquillage bicouche.

La deuxième composition peut également constituer une couche de base appliquée sur la matière kératinique et la première composition une couche de dessus.

En particulier la couche de base est un rouge à lèvres, un fond de teint, un mascara, un brillant à lèvres, un eye liner, un vernis à ongles, un produit de soin pour les ongles, un produit de maquillage du corps, et la couche du dessus un produit de soin ou de protection.

L'invention se rapporte aussi à un procédé de maquillage de la peau et/ou des lèvres et/ou des phanères consistant à appliquer sur la peau et/ou les lèvres et/ou les phanères un produit cosmétique tel que défini précédemment.

L'invention a encore pour objet un procédé cosmétique de soin ou de maquillage de la peau et/ou des lèvres et/ou des phanères d'être humain, consistant à appliquer sur la peau, les lèvres et/ou les phanères une première couche d'une première composition comprenant un polymère siliconé tel que décrit précédemment, puis à appliquer, sur tout ou partie de la première couche, une seconde couche d'une seconde composition comprenant un milieu cosmétiquement acceptable.

L'invention a encore pour objet un procédé cosmétique de soin ou de maquillage de la peau et/ou des lèvres et/ou des phanères d'être humain, consistant à appliquer sur la peau, les lèvres et/ou les phanères une première couche d'une première composition comprenant un milieu cosmétiquement acceptable, puis à appliquer, sur tout ou partie de la première couche, une seconde couche d'une seconde composition comprenant un polymère siliconé tel que décrit précédemment.

Le produit selon l'invention peut être appliqué sur la peau aussi bien du visage que du cuir chevelu et du corps, des lèvres, de l'intérieur des paupières inférieures, et des phanères comme les ongles, les cils, les cheveux, les sourcils, voire les poils. La seconde composition peut former des motifs et peut être appliquée avec un stylo, crayon ou tout autre instrument (éponge, doigt, pinceau, brosse, plume). Ce maquillage peut aussi être appliqué sur les accessoires de maquillage comme les faux ongles, faux cils, perruques ou encore des pastilles ou des patchs adhérents sur la peau ou les lèvres (du type mouches).

L'invention se rapporte également à l'utilisation cosmétique du produit cosmétique défini ci-dessus pour améliorer les propriétés de confort, et/ou de brillance et/ou de non transfert et/ou de non migration et/ou de tenue de la couleur du maquillage, sur la peau et/ou les lèvres et/ou les phanères.

L'invention a enfin pour objet l'utilisation d'un produit cosmétique comprenant une première et une seconde compositions, la première composition comprenant un polymère siliconé tel que décrit ci-après, et la seconde composition comprenant un milieu cosmétiquement acceptable, pour conférer à la peau et/ou les lèvres et/ou les phanères un rendu cosmétique confortable, brillant, non transfert, non migrant, et de bonne tenue de la couleur.

Première composition

La première composition selon l'invention comprend un polymère siliconé particulier comprenant au moins un motif polyhydroxylé.

introduit en quantité suffisante, polymère siliconé présente l'avantage d'améliorer la tenue tout en maintenant la brillance.

Des polymères de silicone utilisables dans la première composition, sont décrits en détail dans la demande de brevet EP 1 213 316, incorporée dans la présente demande par référence.

En particulier, les polymères de silicone utilisables dans la première composition sont représentés par la formule générale (I) suivante:

$$R^1_a\ R^2_b\ R^3_c\ SiO_{(4-a-b-c)/2} \qquad (I)$$

dans laquelle :

a) a, b et c sont tels que a varie de 1 à 2,5 ; et b et c, indépendamment l'un de l'autre, varient de 0,001 à 1,5.
b) $R^1$, identique ou différent, est choisi parmi :

- les radicaux alkyle en $C_1$ à $C_{30}$, le cas échéant substitués par un ou plusieurs atomes de fluor, groupements amino et/ou carboxy,
- les radicaux aryle, aralkyle,
- les radicaux de formule générale (II) :

$$-C_dH_{2d}-O-(C_2H_4O)_e(C_3H_6O)_fR^4 \qquad (II)$$

dans laquelle :

- $R^4$ est un radical hydrocarboné en $C_1$ à $C_{30}$, ou un radical $R^5$-(CO)- avec $R^5$ étant un radical hydrocarboné en $C_1$ à $C_{30}$, et
- d, e et f sont des entiers tels que d varie de 0 à 15, e et f, indépendamment l'un de l'autre, varient de 0 à 50, et

- leurs combinaisons.

c) $R^2$ est représenté par la formule générale suivante (III):

$$-Q-O-X \qquad (III)$$

dans laquelle :

- Q est un radical hydrocarboné bivalent en $C_2$ à $C_{20}$, pouvant inclure au moins une liaison éther et/ou au moins une liaison ester, et
- X est un radical hydrocarboné polyhydroxylé.

d) $R^3$ est un groupement organosiloxane de formule générale (IV) :

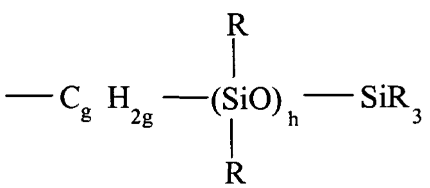

(IV)

avec:

- chacun des radicaux R représentant, indépendamment l'un de l'autre, un radical choisi parmi les radicaux alkyle en $C_1$ à $C_{30}$, le cas échéant substitué par un ou plusieurs atomes de fluor ; et les radicaux aryle et aralkyle,
- g et h étant des entiers tels que g varie de 1 à 5 et h varie de 0 à 500.

Lorsque les radicaux R représentent un radical choisi parmi les radicaux alkyle en $C_1$ à $C_{30}$, le cas échéant substitués par un ou plusieurs atomes de fluor, les radicaux aryle et les radicaux aralkyle, ils ont la même signification que le radical $R^1$ tel que défini précédemment.

Il est à noter que les radicaux $R^1$, $R^2$ et $R^3$ des polymères de silicone de formule générale (I), telle que définie ci-dessus, sont distribués de manière aléatoire ou statistique, c'est-à-dire qu'ils apparaissent dans la structure du polymère sans ordre déterminé. De même, $R^1$, $R^2$, et $R^3$ peuvent figure respectivement des radicaux de nature différente dans un composé de formule générale (I).

Selon un mode de réalisation particulier,

dans a) :

- de manière plus particulière, a varie de 1,2 à 2,3. Et, en particulier, b et c, indépendamment l'un de l'autre, varient de 0,05 à 1.

dans b) :

- lorsque $R^1$ est un radical alkyle, il peut être un radical alkyle en $C_1$ à $C_{30}$, en particulier un radical alkyle en $C_1$ à $C_{25}$, plus particulièrement un radical alkyle en $C_1$ à $C_{20}$, en particulier un radical alkyle en $C_1$ à $C_{10}$, et notamment un radical alkyle en $C_1$ à $C_6$, et en particulier un radical alkyle en $C_1$ à $C_4$. Plus particulièrement, il peut être un radical méthyle, éthyle, n- ou iso-propyle, n- ou iso- ou tert- butyle, pentyle, hexyle, heptyle, octyle, nonyle, décyle, undécyle ou lauryle. Il peut également être un radical cycloalkyle tel qu'un cyclopropyle, un cyclobutyle, cyclopentyle ou un cyclohexyle. Il peut également être un radical alkyle monoinsaturé ou polyinsaturé, linéaire ou ramifié. Il peut également être un radical alkyle substitué par un ou plusieurs atomes de fluor, tel que le trifluoropropyle ou l'heptadécafluorodécyle. Il peut également être un radical alkyle substitué par un ou plusieurs groupements amino, tels que l'amino-2 éthyle, l'amino-3 propyle, le (amino-2 éthyle)amino-3 propyle. Il peut également être un groupe alkyle substitué par un ou plusieurs groupements carboxy, tel que le carboxy-3 propyle.
- $R^1$ peut également être u n radical aryle ou aralkyle tel que le radical phényle, le radical tolyle, le radical benzyle et le radical phénéthyle.
- $R^1$ peut également être un groupe organique représenté par la formule générale (II) :

$$- C_dH_{2d}\text{-}O\text{-}(C_2H_4O)_e(C_3H_6O)_fR^4 \qquad \text{(II)}$$

Selon un mode de réalisation particulier, $R^1$ peut être un radical hydroxylé ou un radical issu de la réaction d'addition d'un alkényléther, saturé ou insaturé, ramifié ou linéaire, dans lequel d = 0 et donc de formule :

$$\text{-}O\text{-}(C_2H_4O)_e(C_3H_6)_fR^4$$

Dans ce cas, lorsque e et f égalent zéro, alors $R^1$ est un groupe alcoxy ayant de 4 à 30 atomes de carbone, par exemple un radical alcoxy inférieur en $C_4$ à $C_{10}$, tel que le butoxy ou le pentoxy ou un radical alcoxy supérieur, en $C_{11}$ à $C_{30}$, tel que l'oléoxy, le stéaroxy, à savoir par exemple, l'alcool cétylique, l'alcool oléique et l'alcool stéarylique, ou un radical issu d'un acide ou d'un acide gras, tel que l'acide acétique, l'acide lactique, l'acide butyrique, l'acide oléique, l'acide stéarique et l'acide béhénique.

Lorsque e et f sont supérieurs à 1, alors $R^1$ est un radical hydroxyle provenant de la réaction d'addition d'un alkylène oxyde.

Lorsque e et f égalent zéro, il est particulièrement avantageux que d soit égal à 3, 5 ou 11. Dans ce cas, $R^1$, selon la nature du substituant R , est un radical allyléther, penthényléther ou undécényléther, ou un radical allylstéaryléther, penthénylbéhényléther, ou undécényloléyléther.

Lorsque e ou f sont différents de zéro, un radical alcoxy et un radical ester sont présents *via* un groupement polyoxyalkylène.

Quelque soit e et f, il est particulièrement avantageux que d soit compris dans l'intervalle variant de 3 à 5.

Selon un mode de réalisation, le radical $R^1$ peut être l'un quelconque des radicaux précédemment définis, ou une combinaison de d eux o u plusieurs de ces radicaux.

De manière avantageuse, $R^1$ est un radical alkyle choisi parmi le radical méthyle, le radical lauryle, et leurs combinaisons.

Par ailleurs, lorsque $R^1$ représente dans la même formule générale (I) deux ou plusieurs radicaux, par exemple un radical méthyle et un radical lauryle, ces réadicaux apparaissent dans la structure de manière aléatoire, et avec

une fréquence qui leur est spécifique.

De manière particulière, au moins 50 % des radicaux $R^1$, en particulier au moins 70 % des radicaux $R^1$, et plus particulièrement 100 % des radicaux $R^1$ sont des radicaux méthyle.

dans c) :

- Q peut être en particulier un radical hydrocarboné bivalent choisi parmi : $-(CH_2)_2-$,$-(CH_2)_3-$, $-CH_2CH(CH_3)-CH_2$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_2-CH(CH_2CH_2CH_3)-$, $-CH_2-CH(CH_2CH_3)-$, $-(CH_2)_3-O-(CH_2)_2-$, $-(CH_2)_3-O-(CH_2)_2-O-(CH_2)_2-$, $-(CH_2)_3-O-CH_2CH(CH_3)-$ et $-CH_2-CH(CH_3)-COO(CH_2)_2-$.
  De manière avantageuse, Q est un radical bivalent choisi parmi $-(CH_2)_2-$ et $-(CH_2)_3-$.
- X peut être de manière particulière un radical hydrocarboné polyhydroxylé comprenant au moins deux résidus hydroxyles, et en particulier un groupement hydrocarboné choisi parmi les dérivés glycérylés et les dérivés osidiques.

Les résidus glycérols peuvent être des composés ayant les formules suivantes, dans lesquelles Q a la même signification que dans la formule générale (III), et s et t sont des entiers compris dans l'intervalle variant de 1 à 20, en particulier de 1 à 15, en particulier de 1 à 10, et plus particulièrement de 1 à 5.

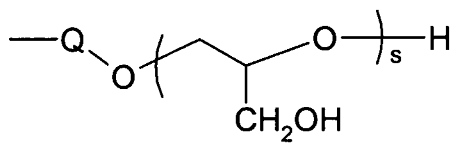

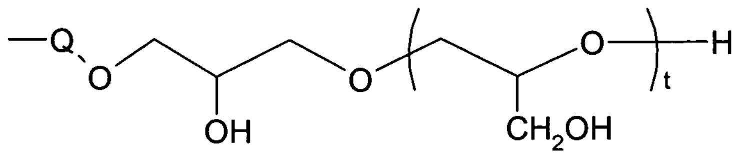

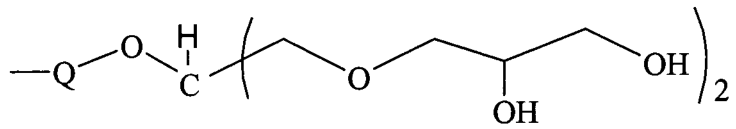

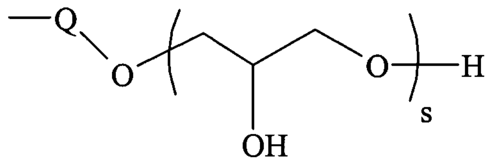

Dans les formules précédentes, un ou plusieurs groupes hydroxyles peuvent être remplacés par des groupes alcoxy ou des groupements esters.

Les radicaux osidiques utilisables dans la formule générale (III) peuvent être de type monosaccharidique, tel que les groupements glycosyle, mannosyle, galactosyle, ribosyle, arabinosyle, xylosyle ou fructosyle, de type oligosaccharidique tel que le maltosyle, le cellobiosyle, le lactosyle ou le maltotriosyle, ou de type polysaccharidique tel que la cellulose ou l'amidon.

De manière particulière, les groupements osidiques sont de type monosaccharidique ou oligosaccharidique.

dans d) :

- chacun des radicaux R peut représenter en particulier, indépendamment l'un de l'autre, un radical choisi parmi les radicaux alkyle en de $C_1$ à $C_{20}$, plus particulièrement en $C_1$ à $C_{10}$, en particulier en $C_1$ à $C_6$, le cas échéant substitué par un ou plusieurs atomes de fluor. Lorsque les radicaux R représentent un radical choisi parmi les radicaux alkyle tels que définis précédemment, le cas échéant substitués par un ou plusieurs atomes de fluor, ils ont la même signification que le radical $R^1$ tel que défini précédemment.
- g, selon un mode particulier de réalisation, est égal à 2

- h, selon un mode particulier de réalisation, est compris dans l'intervalle variant de 1 à 50.

Toutefois, parmi les aspects particuliers de la présente invention, définis précédemment, et notamment selon le septième aspect, le polymère de silicone de formule générale (I) est tel que lorsque le radical $R^2$ est représenté par la formule générale (IIIA) :

$$-C_3H_6O[CH_2CH(OH)CH_2O]_nH \qquad \text{(IIIA)}$$

dans laquelle n varie de 1 à 5, alors le radical $R^1$ est différent d'un radical alkyle en $C_{12}$.

Selon un mode particulier de réalisation, le polymère de silicone de formule générale (I) convenant à la mise en oeuvre de la présente invention est tel que :

- a varie de 1 à 1,4, b et c, indépendamment l'un de l'autre, varient de 0,02 à 0,03, et
- $R^1$ est un radical alkyle en $C_1$ à $C_{10}$, en particulier en $C_1$ à $C_6$, et plus particulièrement en $C_1$ à $C_4$.
- $R^2$ est représenté par la formule (IIIA) :

$$C_3H_6O[CH_2CH(OH)CH_2O]_nH \qquad \text{(IIIA)}$$

dans laquelle :

- n varie de 1 à 5, et
- $R^3$ est représenté par la formule (IVA) :

$$-C_2H_4(CH_3)_2SiO[(CH_3)_2SiO]_mSi(CH_3)_3 \qquad \text{(IVA)}$$

dans laquelle :

- m varie de 3 à 9.

Selon un autre mode particulier de réalisation, le polymère de silicone de formule générale (I), utilisable dans la première composition, est tel que :

- a varie de 1 à 1,4, b et c, indépendamment l'un de l'autre, varient de 0,02 à 0,04,
- $R^1$ est un radical méthyle,
- $R^2$ est représenté par la formule (IIIA) dans laquelle n varie de 1 et 5, et
- $R^3$ est représenté par la formule (IVA) dans laquelle m varie de 3 à 9.

De manière avantageuse, le polymère de silicone de formule générale (I) utilisé dans la première composition, peut être choisi parmi la diméthicone de polyglycéryl-3 polyméthylsiloxyéthyle, la diméthicone de laurylpolyglycéryl-3 polyméthylsiloxyéthyle et la diméthicone de polyglycéryl-3 disiloxane, dont les formules sont respectivement :

- Diméthicone de polyglycéryl-3 polyméthylsiloxyéthyle (formule (V)) :

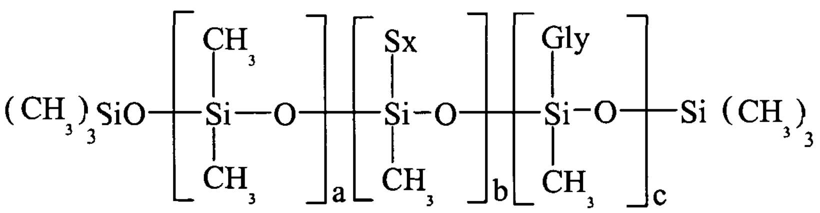

(V)

dans laquelle :

Sx : $-C_2H_4\ [(CH_3)_2SiO]_mSi(CH_3)_3$
Gly : $-C_3H_6O[CH_2\text{-}CH(OH)CH_2O]_nH$

avec a = 1-1,4, b = 0,02-0,04, c = 0,02-0,04, m = 3-9, n = 1-5

- Diméthicone de lauryl polyglycéryl-3 polyméthylsiloxyéthyle (formule (VI)) :

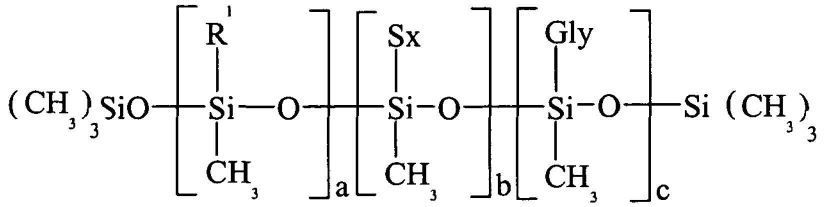

(VI)

dans laquelle Sx, Gly, a, b, c, m et n ont la même signification que précédemment et $R_1$ est, soit un radical méthyle, soit un radical lauryle.

- Diméthicone de polyglycéryl-3 disiloxane (formule (VII)) :

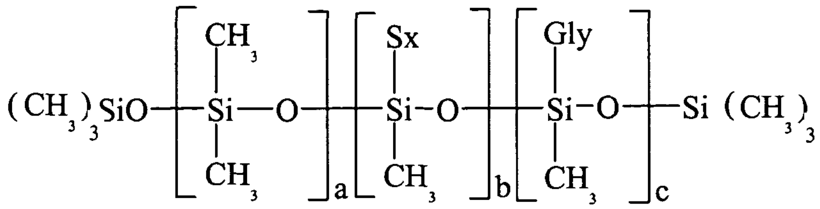

(VII)

dans laquelle Gly, a, b, c, m et n ont la même signification que précédemment, et

Sx : $-O(CH_3)_2SiO-Si(CH_3)_3$

Le polymère de silicone de formule générale (I) peut être présent dans la première composition à raison de 0,1 à 40 % en poids, en particulier de 0,5 à 30 % en poids, plus particulièrement de 1 à 25 % en poids, en particulier de 5 à 20 % en poids, en particulier de 7 à 15 % en poids, par rapport au poids total de la composition.

Selon un mode particulier de réalisation, le polymère de silicone de formule générale (I) est avantageusement choisi parmi les polymères commercialisés par la société SHIN-ETSU sous les références KF6100®, KF6104® et KF6105®.

Selon encore un autre mode de réalisation, le composé désigné sous la référence KF6104®, commercialisé par la société SHIN-ETSU, convient particulièrement pour préparer un produit cosmétique conforme à l'invention présentant une tenue et, éventuellement, une brillance moyenne améliorées.

Par « tenue», on entend la propriété du produit cosmétique selon l'invention à transférer dans une moindre mesure sur des objets avec lesquels il peut entrer en contact, et/ou la propriété à résister à l'interaction avec des liquides, comme les larmes, la sueur, ou le contact avec des aliments lors d'un repas dans le cas d'un rouge à lèvres par exemple et/ou la propriété à ne pas migrer lors du tracé initial du maquillage, en particulier dans le cas des rouges à lèvres, dans les rides et ridules du contour des lèvres.

L'amélioration de la tenue du produit selon l'invention peut donc provenir de l'amélioration de l'une au moins de ces propriétés. Par exemple, l'amélioration de la tenue peut provenir de l'amélioration de son non transfert et/ou de l'amélioration de la tenue de sa couleur.

Le produit cosmétique selon l'invention présente l'avantage de ne pas transférer, du moins en partie, c'est-à-dire de ne laisser que de moindres traces sur certains supports avec lesquels il peut être mise en contact et notamment un verre, une tasse, une cigarette, un mouchoir, un vêtement ou la peau. Le transfert d'une composition cosmétique provoque une mauvaise tenue du film appliqué, nécessitant de renouveler régulièrement l'application de la composition.

Le produit cosmétique selon l'invention présente en outre une bonne tenue de sa couleur. Le film de produit appliqué sur la peau, les lèvres et/ou les phanères peut généralement être altéré lors du contact avec des liquides, notamment de l'eau ou des boissons consommées par exemple lors d'un repas, ou bien encore des huiles comme les huiles alimentaires ou bien encore le sébum ou bien encore de la salive. La tenue de la couleur peut ainsi être caractérisée par la tenue de sa couleur à l'eau et/ou la tenue de sa couleur à l'huile.

Ainsi, l'évaluation des propriétés d e tenue du produit cosmétique selon l'invention peut être caractérisée par la mesure d'au moins l'un des trois paramètres suivants : le non transfert, la tenue de la couleur à l'eau et la tenue de la couleur à l'huile.

On peut mesurer les trois paramètres les uns à la suite des autres selon le protocole décrit ci-après.

Les mesures sont réalisées, par exemple, sur la face interne de l'avant-bras, lavée et laissée séchée naturellement à température ambiante pendant 5 minutes. La première composition cosmétique à tester, par exemple un rouge à lèvres, est appliquée sur trois zones de la face interne de l'avant-bras. On laisse sécher la première composition le temps nécessaire pour que les solvants volatiles éventuellement présents se soient volatilisés, puis on applique la deuxième composition sur la première. La surface de peau sur laquelle les mesures sont réalisées doit être au moins supérieure à 1 $cm^2$. De manière générale, les mesures se font sur des zones circulaires de diamètre environ égal à 3 cm.

Il est nécessaire qu'environ la même quantité de produit cosmétique soit appliquée sur chacune des trois zones. Cela peut être vérifié en mesurant le poids de la première et de la deuxième composition cosmétique, après chacune des applications ou en préparant à l'avance des quantités équivalentes d'échantillon à tester. De manière générale, pour une surface de 1 $cm^2$, une quantité égale à environ 2 mg est nécessaire (si la surface possède un diamètre de 3 cm, alors une quantité d'environ 28 mg est requise).

Après application du produit cosmétique, la couleur, $L_1{}^*a_1{}^*b_1{}^*$, est mesurée en chacune des trois zones, et la valeur moyenne obtenue correspond à la couleur initiale du produit. La mesure de la couleur peut être effectuée avec un colorimètre MINOLTA de la série CR200 ou CR300 ou CM500 ou CM1000 ou CM2000. On utilise en particulier le colorimètre MINOLTA de la série CR200.

On ajoute 20 $mg/cm^2$ d'eau sur chacune des trois zones à tester (pour des surfaces dont le diamètre est égal à environ 3 cm, environ 280 mg d'eau doivent être appliqués). Chacune des zones à tester est ensuite soumise à un massage manuel pendant quelques secondes, en particulier de 2 à 5 secondes, et plus particulièrement de 2 secondes.

Une épaisseur d'un mouchoir en papier blanc du commerce tel que Kleenex, dont la couleur $L_0{}^*a_0{}^*b_0{}^*$ a été mesurée, est appliqué chaque zone maquillée pendant environ 5 secondes et à une force d'environ 100 g/f, laquelle force peut être appliquée avec un dynamomètre de pression digital DPZ-5N du constructeur, IMADA co.ltd.

La valeur de transfert T est obtenue par soustraction de la couleur du tissu blanc mesurée avant application sur la zone à tester, $L_0{}^*a_0{}^*b_0{}^*$, et de la couleur moyenne $L_2{}^*a_2{}^*b_2{}^*$ correspondant à la moyenne des valeurs obtenues pour chaque mouchoir après leur application sur chaque zone de l'avant bras recouverte de produit à tester,

On détermine alors la différence de couleur ΔE(T) entre la couleur du mouchoir avant et après son application sur la zone de l'avant bras portant le produit :

$$\Delta E\,(T) = \sqrt{(L_2{}^*-L_o{}^*)^2 + (a_2{}^* - a_o{}^*)^2 + (b_2{}^* - b_o{}^*)^2}$$

Plus la valeur obtenue ΔE (T) est faible, plus le produit cosmétique est considérée comme présentant un bon niveau de non transfert.

De manière avantageuse, le produit cosmétique, selon la présente invention, possèdent une valeur de transfert ΔE (T) inférieure ou égale à 45, en particulier inférieure ou égale à 40, notamment inférieure ou égale à 35, notamment comprise entre 0 et 45.

On mesure ensuite la couleur moyenne $L_3{}^*a_3{}^*b_3{}^*$ du produit après application du mouchoir.

La valeur de tenue de la couleur à l'eau peut être obtenue après réalisation du test de transfert. Elle est par exemple égale à la différence de couleur entre la couleur initiale moyenne $L_1{}^*a_1{}^*b_1{}^*$ du produit appliqué sur l'avant-bras et de la couleur $L_3{}^*a_3{}^*b_3{}^*$ moyenne de la zone de l'avant-bras portant le produit après application de l'eau et du mouchoir

$$\Delta E\,(E) = \sqrt{(L_3{}^*-L_1{}^*)^2 + (a_3{}^* - a_1{}^*)^2 + (b_3{}^* - b_1{}^*)^2}$$

Plus la valeur obtenue est faible, plus le produit cosmétique est considérée comme possédant une bonne tenue de la couleur à l'eau.

Avantageusement, les produits cosmétiques selon la présente invention, possèdent u ne valeur de tenue de la couleur à l'eau inférieure o u é gale à 15, e n particulier inférieure ou égale à 10, et plus particulièrement inférieure ou égale à 6. De manière avantageuse, la valeur de tenue de la couleur varie 0 à 15.

Le test de tenue de la couleur à l'huile est conduit par application sur les zones à tester d'environ 20 $mg/cm^2$ d'huile de type alimentaire sur chaque zone de l'avant-bras (huile de colza, huile de soja ou huile de tournesol) suivi d'un massage manuel pendant quelques secondes, en particulier pendant 2 à 5 secondes, et plus particulièrement

pendant 2 secondes. Puis une épaisseur d'un mouchoir en papier blanc du commerce tel qu'un mouchoir Kleenex est appliquée sur la zone pendant environ 5 secondes et à une force d'environ 100 g/f, laquelle force peut être appliquée avec un dynamomètre de pression digital DPZ-5N du constructeur, IMADA Co.Ltd.

La valeur de tenue de la couleur à l'huile, H, est égale à la différence entre la couleur moyenne $L_4{}^*a_4{}^*b_4{}^*$ du produit restant sur l'avant bras après le massage à l'huile et l'application du mouchoir, et la couleur moyenne $L_1{}^*a_1{}^*b_1{}^*$ mesurée initialement,

$$\Delta E\ (H) = \sqrt{(L_4{}^* - L_1{}^*)^2 + (a_4{}^* - a_1{}^*)^2 + (b_4{}^* - b_1{}^*)^2}$$

Le test de tenue de la couleur à l'huile est un test permettant notamment d'évaluer le maintien d'une composition cosmétique telle qu'un rouge à lèvres lors d'un repas.

De manière avantageuse, les produits cosmétiques, selon la présente invention, possèdent une valeur de tenue de la couleur à l'huile inférieure ou égale à 25, inférieure ou égale à 10, inférieure ou égale à 8. En particulier, la valeur de tenue de la couleur à l'huile varie de 0 à 25.

De façon encore plus avantageuse, le produit selon l'invention possède une valeur de transfert inférieure ou égale à 35, une valeur de tenue de la couleur à l'eau inférieure à 6, et une valeur de tenue de la couleur à l'huile inférieure ou égale à 8.

Avantageusement, le polymère de silicone de formule générale (I) est tel, que lorsqu'il est en quantité suffisante dans la première composition, la brillance moyenne à 60° d'un dépôt du produit selon l'invention, une fois étalée sur un support, est égale ou supérieure à 30 sur 100.

Par "brillance moyenne", on désigne la brillance telle qu'elle peut être mesurée à l'aide d'un brillancemètre, de manière conventionnelle par la méthode suivante.

Un brillancemètre de type MINOLTA GM268 peut être utilisé. Les mesures sont réalisées sur des zones tests de surface supérieure à 1 $cm^2$. De manière standard, les surfaces sur lesquelles est étalé le produit à tester font environ 2,5 cm x 4 cm.

La première composition cosmétique à évaluer est appliquée sur une surface synthétique, de type BIOSKIN. La quantité de composition cosmétique à appliquer est d'environ 1 $mg/cm^2$. On laisse sécher la première composition puis on applique environ 1 $mg/cm^2$ de deuxième composition.

La valeur de la brillance est obtenue par mesure de la réflectance à un angle d'environ 60°.

Cinq mesures par échantillon sont nécessaires, les deux mesures présentant les valeurs les plus élevées et la mesure présentant la valeur la plus basse sont écartées, et une moyenne est réalisée sur les trois valeurs mesurées restantes.

La brillance moyenne du produit cosmétique selon la présente invention est avantageusement supérieure ou égale à 30, en particulier supérieure ou égale à 40, et plus particulièrement supérieure ou égale à 45.

Le confort du produit selon l'invention est évalué selon le test décrit ci-après. Dans ce test, le confort est mesuré par un test de traction d'une bandelette de latex. Ce test prévoit la capacité, pour un dépôt produit, de résister à l'écaillage et au pelage susceptible de survenir à la suite des les mouvements de la peau.

Des échantillons de première composition cosmétique sont appliqués sur des surfaces de, par exemple, 2,54 x 2,54 cm, d'une bande de latex de 2,54 cm de large, obtenue, par exemple, par découpe dans la zone du poignet d'un gant de type ANSELL EDMOND INDUSTRIAL TECHNICIANS REF#390, taille 9.

La quantité de la première composition cosmétique à déposer est telle que le poids en matière sèche de la composition doit être d'environ 20 mg.

On laisse sécher la première composition, puis on applique la deuxième dans les mêmes conditions que précédemment.

La première et la deuxième composition sont successivement appliquées sur la bande de latex à l'aide d'un pinceau à lèvres jetable, par exemple du type de ceux produits par la société FEMME COSMETICS, INC., LA.

La quantité de deuxième composition cosmétique à déposer est telle que le poids en matière sèche de la composition doit être d'environ 20 mg.

Les échantillons ainsi préparés sont laissés pendant 24 heures à température ambiantes.

Puis, le poids de la bande de latex comprenant le dépôt du produit cosmétique est mesuré (B). La soustraction de la valeur du poids de la bande de latex dépourvue de composition cosmétique (A) de la valeur ainsi mesurée (B) doit correspondre au poids de film sec, et donc être d'environ 40 $\pm$ 2 mg.

La bande de latex portant les échantillons de composition cosmétique à tester est ensuite étirée de telle manière que la zone supportant l'échantillon doit atteindre la longueur de 4,445 cm (1,75 pouce).

Les fragments de film de la composition cosmétique détachés sur la bande de latex sont observés, puis éliminés par balayage à l'aide du pinceau à lèvres.

Le poids de la bande de latex comprenant la composition cosmétique restante, est ensuite mesuré (D).

Le pourcentage de perte de poids du film de la composition cosmétique est ensuite calculé à l'aide de l'équation suivante :

$$\text{Indice de confort} = [(D-A)/(B-A)] \times 100.$$

Les mesures sont répétées trois fois pour chaque composition cosmétique testée. L'indice de confort de la composition selon l'invention est égal à la moyenne de ces trois mesures.

Avantageusement, le polymère de silicone de formule générale (I) est tel, que lorsqu'il est en quantité suffisante dans la composition cosmétique l'indice de confort d'un dépôt du produit, une fois étalée sur un support, est égale ou supérieure à 80 sur 100, de préférence supérieur à 90 sur 100, de préférence supérieur à 95 sur 100.

De manière avantageuse, la première composition peut comprendre au moins un agent filmogène, associé ou non avec au moins un auxiliaire de filmification.

Le polymère filmogène additionnel peut être un des polymères décrits dans la demande FR0450540 déposée le 18 mars 2004, dont le contenu est inclus dans la présente demande par référence.

Le polymère filmogène additionnel présent dans la composition selon l'invention en une teneur allant de 0,1 % à 60 % en poids, par rapport au poids total de la composition, de préférence allant de 2 % à 40 % en poids, et mieux de 5 % à 25 % en poids.

Par « polymère filmogène », on désigne un polymère apte à former à lui seul ou en présence d'un agent auxiliaire de filmification, un film isolable, notamment continu et adhérant sur un support, notamment sur les matières kératiniques, et de préférence un film cohésif et mieux encore, un film dont la cohésion et les propriétés mécaniques sont telles que ledit film peut être isolé dudit support.

Avantageusement, l'agent filmogène est présent, dans les compositions cosmétiques conformes à l'invention, dans un rapport pondéral par rapport au poids des polymères de silicone de formule générale (I), inférieur à 5:1, en particulier inférieur à 1:1, et plus particulièrement inférieur ou égal à 1:2.

Comme polymères filmogènes convenant particulièrement à l'invention, on peut citer les polymères et copolymères acryliques, les polyuréthannes, les polyesters, les polyamides, les polyurées, les polymères cellulosiques comme la nitrocellulose, et les polymères siliconés.

Il est également avantageux d'utiliser des résines de silicone, qui sont des polymères de polyorganosilixaves réticulés généralement solubles ou gonflables dans les huiles de silicones.

A titre d'exemple de ces résines de silicone, on peut citer les siloxysilicates, les polysilesquioxanes et les polyméthylsesquioxanes.

A titre d'exemple de résines polyméthylsesquioxanes commercialement disponible, on peut citer celles qui sont commercialisées par la société WACKER sous la référence RESIN MK, tel que la BELSIL PMS MK ; celles commercialisées par la société SHIN-ETSU, par exemple sous les références KR-220L.

Comme résines siloxysilicates, on peut citer les résines triméthylsiloxysilicates commercialisées sous la référence SR1000, par la société GENERAL ELECTRIC, ou sous la référence TMS803 par la société WACKER. On peut également citer les résines triméthylsiloxysilicates commercialisées sous la dénomination KF-7312J par la société SHIN-ETSU, et les résines DC749, DC593 commercialisées par la société DOW CORNING.

Comme polymères siliconés liposolubles, on peut également mentionner les polyamides siliconés du type polyorganosiloxane, tels que ceux décrits dans les documents US 5 874 069, US 5 919 441, US 6 051 216 et US 5 981 680.

Selon un mode de réalisation particulier, un polymère filmogène siliconé convenant particulièrement à la mise en oeuvre de la présente invention, peut être un copolymère comportant des groupements carboxylates et des groupements polydiméthylsiloxanes.

Par « copolymère comportant des groupements carboxylates et des groupements polydiméthylsiloxanes », on entend dans la présente demande, un copolymère obtenu à partir de (a) un ou plusieurs monomères carboxyliques (acide ou ester), et (b) une ou plusieurs chaînes polydiméthylsiloxane (PDMS).

On entend dans la présente demande par « monomère carboxylique » aussi bien les monomères d'acide carboxylique que les monomères d'ester d'acide carboxylique. Ainsi, le monomère (a) peut être choisi par exemple parmi l'acide acrylique, l'acide méthacrylique, l'acide maléique, l'acide fumarique, l'acide itaconique, l'acide crotonique, leurs esters et les mélanges de ces monomères. Comme esters, on peut citer les monomères suivants : acrylate, méthacrylate, maléate, fumarate, itaconoate et/ou crotonoate. Les monomères sous forme d'esters sont plus particulièrement choisis parmi les acrylates et méthacrylates d'alkyle linéaire ou ramifié de préférence en $C_1$-$C_{24}$ et mieux en $C_1$-$C_{22}$, le radical alkyle étant préférentiellement choisi parmi les radicaux méthyle, éthyle, stéaryle, butyle, éthyl-2-hexyle,

et leurs mélanges.

Le copolymère peut comprendre comme groupements carboxylates, au moins un groupement choisi parmi l'acide acrylique, l'acide méthacrylique, les acrylates ou méthacrylates de méthyle, d'éthyle, de stéaryle, de butyle, d'éthyl-2-hexyle, et leurs mélanges.

On entend désigner par « polydiméthylsiloxanes » (appelé aussi organopolysiloxanes ou, en abréviation, PDMS), en conformité avec l'acceptation générale, tout polymère ou oligomère organosilicié à structure linéaire, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), comportant des radicaux triméthyle directement liés par l'intermédiaire d'un atome de carbone sur lesdits atomes de silicium. Les chaînes PDMS pouvant être utilisées pour obtenir le copolymère comportent au moins un groupe radical polymérisable, de préférence situé sur au moins l'une des extrémités de la chaîne, c'est-à-dire que le PDMS peut avoir par exemple un groupe radical polymérisable sur les deux extrémités de la chaîne ou avoir un groupe radical polymérisable sur une extrémité de la chaîne et un groupement terminal triméthylsilyle sur l'autre extrémité de la chaîne. Le groupe radical polymérisable peut être notamment un groupe acrylique ou méthacrylique, en particulier un groupe $CH_2= CR_1 - CO - O - R_2$, où $R_1$ représente un hydrogène ou un groupe méthyle, et $R_2$ représente $-CH_2-$, $-(CH_2)_n-$ avec n = 3, 5, 8 ou 10, $-CH_2CH(CH_3)-CH_2-$ , $-CH_2-CH_2O-CH_2-CH_2-$, $-CH_2-CH_2O-CH_2CH_2-CH(CH_3)-CH_2$, $-CH_2CH_2-O-CH_2\ CH_2-O-CH_2-CH_2-CH_2-$.

Les copolymères utilisés sont généralement obtenus selon les méthodes usuelles de polymérisation et de greffage, par exemple par polymérisation radicalaire (A) d'un PDMS comportant au moins un groupe radical polymérisable (par exemple sur l'une des extrémités de la chaîne ou sur les deux) et (B) d'au moins un monomère carboxylique, comme décrit par exemple dans les documents US-A-5,061,481 et US-A-5,219,560.

Les copolymères obtenus ont généralement un poids molécule allant d'environ 3000 à 200 000 et de préférence d'environ 5000 à 100 000.

Le copolymère peut se présenter tel quel ou sous forme dispersée dans un solvant tel que les alcools inférieurs comportant de 2 à 8 atomes de carbone, comme l'alcool isopropylique, ou les huiles comme les huiles de silicone volatiles (par exemple cyclopentasiloxane).

Comme copolymères utilisables, on peut citer par exemple les copolymères d'acide acrylique et d'acrylate de stéaryle à greffons polydiméthylsiloxane, les copolymères de méthacrylate de stéaryle à greffons polydiméthylsiloxane, les copolymères d'acide acrylique et de méthacrylate de stéaryle à greffons polydiméthylsiloxane, les copolymères de méthacrylate de méthyle, méthacrylate de butyle, d'acrylate d'éthyl-2-hexyle et de méthacrylate de stéaryle à greffons polydiméthylsiloxane. On peut citer en particulier comme copolymère utilisable, l es copolymères commercialisés par la société SHIN-ETSU sous les dénominations KP-561 (nom CTFA : acrylates/dimethicone), KP-541 où le copolymère est dispersé à 60 % en poids dans de l'alcool isopropylique (nom CTFA : acrylates/dimethicone and Isopropyl alcohol), KP-545 où le copolymère est dispersé à 30 % dans du cyclopentasiloxane (nom CTFA : acrylates/dimethicone and Cyclopentasiloxane). Selon un mode préféré de réalisation de l'invention, on utilise de préférence le KP561 ; ce copolymère n'est pas dispersé dans un solvant, mais se présente sous forme cireuse, son point de fusion étant d'environ 30°C.

Seconde composition

Le produit cosmétique selon l'invention comprend une seconde composition contenant un milieu cosmétiquement acceptable.

Par « milieu physiologiquement acceptable », o n désigne un milieu non toxique et susceptible d'être appliqué sur la peau ou les lèvres d'êtres humains. Le milieu physiologiquement acceptable est généralement adapté à la nature du support sur lequel doit être appliquée la composition, ainsi qu'à l'aspect sous lequel la composition est destinée à être conditionnée.

Le milieu physiologiquement acceptable peut comprendre une phase aqueuse et/ou une phase grasse.

Selon un mode particulier de réalisation, la phase aqueuse ou la phase grasse peut former la phase continue de la composition.

La seconde composition est avantageusement choisie de telle manière qu'elle améliore au moins une propriété cosmétique de la première composition, lorsque celle-ci est appliquée seule sur ladite matière kératinique, sans pour autant dégrader une des autres propriétés cosmétiques de ladite première composition.

Selon un mode de mise en oeuvre, la deuxième composition permet d'améliorer la brillance, ainsi que la tenue de la brillance, de la première composition.

Selon un premier mode de mise en oeuvre, la première composition contient majoritairement en poids, voir est constituée de composés hydrocarbonés, et la deuxième composition contient majoritairement en poids, voir et constituée de composés siliconés. Les termes siliconés et hydrocarbonés ont la même signification que précédemment. Par « contient majoritairement » on entend contient au moins 50% en poids, et mieux au moins 60% en poids, mieux encore au moins 70% en poids, mieux encore au moins 80% en poids, mieux encore au moins 90% en poids, et mieux

encore au moins 95% en poids.

Selon un deuxième mode de réalisation, la première composition contient majoritairement en poids des composés, voir est constituée de composés siliconés, et la deuxième composition contient majoritairement en poids, voir et constituée de composés hydocarbonés.

Selon un mode de réalisation, la deuxième composition est transparente.

On entend par « composition transparente », une composition transparente à translucide, c'est-à-dire telle qu'elle transmet au moins 40 % de lumière à une longueur d'onde de 750 nm, et de préférence au moins 50 % de lumière. La mesure de la transmission est effectuée avec un spectrophotomètre UV-visible Cary 300 Scan de la société Varian selon le protocole suivant :

On coule la composition au-dessus de sa température de fusion dans une cuve à spectrophotomètre à section carrée dont le côté a une longueur de 10 mm.

L'échantillon de la composition est ensuite refroidi pendant 24 heure à 35 °C puis maintenu dans une enceinte thermostatée à 20°C pendant 24 heures.

On mesure ensuite la lumière transmise à travers l'échantillon de la composition au spectrophotomètre par balayage de longueurs d'onde allant de 700 nm à 800 nm, la mesure étant faite en mode transmission.

On détermine alors le pourcentage de lumière transmise à travers l'échantillon de la composition à la longueur d'onde de 750 nm.

Lorsque la seconde composition est transparente, elle contient avantageusement moins de 5% de pigments, de préférence moins de 2%, de préférence encore moins de 1 %.

De manière avantageuse, la première et/ou la deuxième composition peut comprendre indépendamment l'une de l'autre, au moins un agent filmogène, associé ou non avec au moins un auxiliaire de filmification.

Le polymère filmogène additionnel peut être un des polymères décrits dans la demande FR0450540 déposée le 18 mars 2004, dont le contenu est inclus dans la présente demande par référence.

Le polymère filmogène additionnel peut être présent dans la première ou deuxième composition selon l'invention en une teneur allant de 0,1 % à 60 % en poids, par rapport au poids total de la composition, de préférence allant de 2 % à 40 % en poids, et mieux de 5 % à 25 % en poids.

Par « polymère filmogène », on désigne un polymère apte à former à lui seul ou en présence d'un agent auxiliaire de filmification, un film isolable, notamment continu et adhérant sur un support, notamment sur les matières kératiniques, et de préférence un film cohésif et mieux encore, un film dont la cohésion et les propriétés mécaniques sont telles que ledit film peut être isolé dudit support.

Avantageusement, l'agent filmogène est présent, dans la première ou deuxième composition, dans un rapport pondéral par rapport au poids des polymères de silicone de formule générale (I), inférieur à 5:1, en particulier inférieur à 1:1, et plus particulièrement inférieur ou égal à 1:2.

Comme polymères filmogènes convenant particulièrement à l'invention, on peut citer les polymères et copolymères acryliques, les polyuréthannes, les polyesters, les polyamides, les polyurées, les polymères cellulosiques comme la nitrocellulose, et les polymères siliconés.

Il est également avantageux d'utiliser des résines de silicone, qui sont des polymères de polyorganosilixaves réticulés généralement solubles ou gonflables dans les huiles de silicones.

A titre d'exemple de ces résines de silicone, on peut citer les siloxysilicates, les polysilesquioxanes et les polyméthylsesquioxanes.

A titre d'exemple de résines polyméthylsesquioxanes commercialement disponible, on peut citer celles qui sont commercialisées par la société WACKER sous la référence RESIN MK, tel que la BELSIL PMS MK ; celles commercialisées par la société SHIN-ETSU, par exemple sous les références KR-220L.

Comme résines siloxysilicates, on peut citer les résines triméthylsiloxysilicates commercialisées sous la référence SR1000, par la société GENERAL ELECTRIC, ou sous la référence TMS803 par la société WACKER. On peut également citer les résines triméthylsiloxysilicates commercialisées sous la dénomination KF-7312J par la société SHIN-ETSU, et les résines DC749, DC593 commercialisées par la société DOW CORNING.

Comme polymères siliconés liposolubles, on peut également mentionner les polyamides siliconés du type polyorganosiloxane, tels que ceux décrits dans les documents US 5 874 069, US 5 919 441, US 6 051 216 et US 5 981 680.

Selon un mode de réalisation particulier, un polymère filmogène siliconé convenant particulièrement à la mise en oeuvre de la présente invention, peut être un copolymère comportant des groupements carboxylates et des groupements polydiméthylsiloxanes.

Par « copolymère comportant des groupements carboxylates et des groupements polydiméthylsiloxanes », on entend dans la présente demande, un copolymère obtenu à partir de (a) un ou plusieurs monomères carboxyliques (acide ou ester), et (b) une ou plusieurs chaînes polydiméthylsiloxane (PDMS).

On entend dans la présente demande par « monomère carboxylique » aussi bien les monomères d'acide

carboxylique que les monomères d'ester d'acide carboxylique. Ainsi, le monomère (a) peut être choisi par exemple parmi l'acide acrylique, l'acide méthacrylique, l'acide maléique, l'acide fumarique, l'acide itaconique, l'acide crotonique, leurs esters et les mélanges de ces monomères. Comme esters, on peut citer les monomères suivants : acrylate, méthacrylate, maléate, fumarate, itaconoate et/ou crotonoate. Les monomères sous forme d'esters sont plus particulièrement choisis parmi les acrylates et méthacrylates d'alkyle linéaire ou ramifié de préférence en $C_1$-$C_{24}$ et mieux en $C_1$-$C_{22}$, le radical alkyle étant préférentiellement choisi parmi les radicaux méthyle, éthyle, stéaryle, butyle, éthyl-2-hexyle, et leurs mélanges.

Le copolymère peut comprendre comme groupements carboxylates, au moins un groupement choisi parmi l'acide acrylique, l'acide méthacrylique, les acrylates ou méthacrylates de méthyle, d'éthyle, de stéaryle, de butyle, d'éthyl-2-hexyle, et leurs mélanges.

On entend désigner par « polydiméthylsiloxanes » (appelé aussi organopolysiloxanes ou, en abréviation, PDMS), en conformité avec l'acceptation générale, tout polymère ou oligomère organosilicié à structure linéaire, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), comportant des radicaux triméthyle directement liés par l'intermédiaire d'un atome de carbone sur lesdits atomes de silicium. Les chaînes PDMS pouvant être utilisées pour obtenir le copolymère comportent au moins un groupe radical polymérisable, de préférence situé sur au moins l'une des extrémités de la chaîne, c'est-à-dire que le PDMS peut avoir par exemple un groupe radical polymérisable sur les deux extrémités de la chaîne ou avoir un groupe radical polymérisable sur une extrémité de la chaîne et un groupement terminal triméthylsilyle sur l'autre extrémité de la chaîne. Le groupe radical polymérisable peut être notamment un groupe acrylique ou méthacrylique, en particulier un groupe $CH_2 = CR_1 - CO - O - R_2$, où $R_1$ représente un hydrogène ou un groupe méthyle, et $R_2$ représente $-CH_2-$, $-(CH_2)_n-$ avec n = 3, 5, 8 ou 10, $-CH_2CH(CH_3)-CH_2-$ , $-CH_2-CH_2-O-CH_2-CH_2-$, $-CH_2-CH_2-O-CH_2-CH_2-CH(CH_3)-CH_2-$, $-CH_2CH_2-O-CH_2$ $CH_2-O-CH_2-CH_2-CH_2-$,

Les copolymères utilisés sont généralement obtenus selon les méthodes usuelles de polymérisation et de greffage, par exemple par polymérisation radicalaire (A) d'un PDMS comportant au moins un groupe radical polymérisable (par exemple sur l'une des extrémités de la chaîne ou sur les deux) et (B) d'au moins un monomère carboxylique, comme décrit par exemple dans les documents US-A-5,061,481 et US-A-5,219,560.

Les copolymères obtenus ont généralement un poids molécule allant d'environ 3000 à 200 000 et de préférence d'environ 5000 à 100 000.

Le copolymère peut se présenter tel quel ou sous forme dispersée dans un solvant tel que les alcools inférieurs comportant de 2 à 8 atomes de carbone, comme l'alcool isopropylique, ou les huiles comme les huiles de silicone volatiles (par exemple cyclopentasiloxane).

Comme copolymères utilisables, on peut citer par exemple les copolymères d'acide acrylique et d'acrylate de stéaryle à greffons polydiméthylsiloxane, les copolymères de méthacrylate de stéaryle à greffons polydiméthylsiloxane, les copolymères d'acide acrylique et de méthacrylate de stéaryle à greffons polydiméthylsiloxane, les copolymères de méthacrylate de méthyle, méthacrylate de butyle, d'acrylate d'éthyl-2-hexyle et de méthacrylate de stéaryle à greffons polydiméthylsiloxane. On peut citer e n particulier comme copolymère utilisable, les copolymères commercialisés par la société SHIN-ETSU sous les dénominations KP-561 (nom CTFA : acrylates/dimethicone), KP-541 où le copolymère est dispersé à 60 % en poids dans de l'alcool isopropylique (nom CTFA : acrylates/dimethicone and Isopropyl alcohol), KP-545 où le copolymère est dispersé à 30 % dans du cyclopentasiloxane (nom CTFA: acrylates/dimethicone and Cyclopentasiloxane). Selon un mode préféré de réalisation de l'invention, on utilise de préférence le KP561 ; ce copolymère n'est pas dispersé dans un solvant, mais se présente sous forme cireuse, son point de fusion étant d'environ 30°C.

La première et la deuxième compositions peuvent comprendre indépendamment l'une de l'autre une phase grasse, notamment constituée de corps gras liquides à température ambiante (20 - 25°C) et pression atmosphérique (huiles) et/ou de cires ou de corps gras pâteux.

La ou les huiles peuvent être présentes à raison de 0,1 à 99 % en poids, en particulier d'au moins 1 à 90 % en poids, plus particulièrement de 5 à 70 % en poids, notamment de 10 à 60 % en poids, voire de 20 à 50 % en poids, par rapport au poids total de la première ou deuxième composition cosmétique.

Les huiles convenant à la préparation des première ou deuxième compositions cosmétiques peuvent être des huiles volatiles ou non, siliconées ou non.

Les huiles volatiles ou non volatiles peuvent être des huiles hydrocarbonées notamment d'origine animale ou végétale, des huiles synthétiques, des huiles siliconées, des huiles fluorées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'ISOPARS® ou de PERMETHYLS®.

Comme huiles volatiles, on peut aussi utiliser les silicones volatiles, comme par exemple les huiles de silicones linéaires ou cycliques volatiles, notamment celles ayant une viscosité ≤ 8 centistokes (8 x $10^{-6}$ $m^2/s$), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des solvants volatils fluorés tels que le nonafluorométhoxybutane ou le perfluorométhylcyclopentane, et leurs mélanges.

Les première ou deuxième compositions cosmétiques selon la présente l'invention peuvent également comprendre indépendamment l'une de l'autre au moins une huile volatile.

Selon un mode particulier de réalisation, les première ou deuxième compositions cosmétiques selon l'invention comprennent moins de 30 % en poids, et en particulier moins de 15 %, en particulier moins de 10 %, et plus particulièrement moins de 5 %, en poids d'huile volatile, par rapport au poids total de la composition.

Selon un autre mode de réalisation, les première ou deuxième compositions cosmétiques sont exemptes d'huiles volatiles.

La première ou deuxième composition cosmétique peut également comprendre au moins une huile non volatile.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :

- les huiles hydrocarbonées d'origine animale,
- les huiles hydrocarbonées d'origine végétale telles que les esters de phytostéaryle, tels que l'oléate de phytostéaryle, l'isostéarate de physostéaryle et le glutanate de lauroyl/octyldodécyle/phytostéaryle (AJINOMOTO, ELDEW PS203), les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment des triglycérides héptanoïques ou octanoïques, les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STEARINERIES DUBOIS ou ceux vendus sous les dénominations MIGLYOL 810®, 812® et 818® par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le Parleam, le squalane et leurs mélanges, et en particulier le polyisobutène hydrogéné,
- les esters hydrocarbonés courts

Au sens de la présente invention, on entend par « ester hydrocarboné court », un ester hydrocarboné comprenant moins de 40 atomes de carbone.

Les première ou deuxième compositions cosmétiques peuvent comprendre indépendamment l'une de l'autre au moins un ester hydrocarboné court.

Les esters conformes à l'invention peuvent être des monoesters, des diesters ou des polyesters et sont plus particulièrement des monoesters c'est-à-dire portant une unique fonction ester. Ces esters peuvent être linéaires, ramifiés ou cycliques, saturés ou insaturés. En particulier, ils sont ramifiés et saturés. Ils peuvent également être volatils ou non volatils.

En particulier, les esters hydrocarbonés peuvent répondre à la formule RCOOR' dans laquelle RCOO représente un reste d'acide gras comportant de 2 à 28 atomes de carbone, et R' représente une chaîne hydrocarbonée contenant de 1 à 28 atomes de carbone. Plus particulièrement les groupements R et R' sont tels que l'ester correspondant est non volatil.

De manière avantageuse, les esters hydrocarbonés mono- di- ou polyesters, utilisables dans les première ou deuxième compositions cosmétiques, comprennent moins de 40 atomes de carbone, et plus de 10 atomes de carbone

Ces esters non volatils peuvent notamment être en $C_{10}$ à $C_{25}$ et en particulier en $C_{14}$ à $C_{22}$. Ils peuvent être choisis parmi des esters des acides en $C_2$ à $C_{18}$ et notamment, des alcools en $C_2$ à $C_{20}$ ou de polyols en $C_2$ à $C_8$ ou de leurs mélanges.

Ils comportent avantageusement moins de 22 atomes de carbone.

Selon un mode de réalisation particulier, lorsque l'ester hydrocarboné comprend moins de 22 atomes de carbone, il

n'est pas une huile volatile.

Ainsi, les esters peuvent être choisis parmi une liste non limitative comprenant les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'octyle, l'isononanoate d'isodécyle, l'isononanoate d'isotridécyle, l'isononanoate d'isostéaryle, mais aussi les esters de l'alcool isopropylique, tels que le myristate d'isopropyle, le palmitate d'isopropyle, le stéarate ou l'isostéarate d'isopropyle, l'octanoate de cétyle, l'octanoate de tridécyle, le 4-diheptanoate et le palmitate d'éthyle 2-hexyle, le benzoate d'alkyle, le diheptanoate de polyéthylène glycol, le diéthyl 2- d'hexanoate de propylèneglycol et leurs mélanges. Ledit ester peut être également choisi parmi les esters de synthèse notamment d'acide gras comme l'huile de purcellin, le myristate d'isopropyle, le palmitate d'éthyle, le stéarate d'octyle ; les esters hydroxylés comme le lactate d'isostéaryle, l'hydroxystéarate d'octyle, l'adipate de diisopropyle, les heptanoates, octanoates, décanoates d'alcool gras et leurs mélanges.

L'isononanoate d'isononyle, l'isononoate d'isotridécyle, l'heptanoate de stéaryle, et leurs mélanges conviennent tout particulièrement à la réalisation de l'invention.

Selon un mode de réalisation particulier, l'ester hydrocarboné court utilisé dans la première composition cosmétique conforme à la présente invention est un mélange d'isononoate d'isononyle, d'isononanoate d'isotridécyle et d'héptanoate de stéaryle.

Cet ou ces ester(s) hydrocarboné(s) peut(peuvent) être utilisé(s) dans la première ou deuxième composition à raison de 5 à 90 %, notamment de 10 à 60 % et en particulier de 20 à 50 % en poids par rapport au poids total de la composition.

- les esters de polyols et les esters du pentaérythritol, comme le tétrahydroxystéarate/tétraisostéarate de dipentaérythritol,
- les esters de dimères diols et dimères diacides tels que les Lusplan DD-DA5 et DD-DA7, décrits dans la demande FR0302809 déposée le 6 mars 2003, dont le contenu est incorporé dans la présente demande par référence.
- les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges, et
- les carbonates de di-alkyle, les 2 chaînes alkyles pouvant être identiques ou différentes, tel que le dicaprylyl carbonate commercialisé sous la dénomination CETIOL CC®, par COGNIS.

Les huiles de silicone non volatiles utilisables dans la première ou deuxième composition peuvent être les polydiméthylsiloxanes (PDMS) non volatiles, comme la siméthicone, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicones de viscosité inférieure ou égale à 100 cSt, et leurs mélanges.

De manière avantageuse, l'huile non volatile hydrocarbonée présente dans la première composition cosmétique, est notamment choisie parmi le polyisobutène hydrogéné, l'heptanoate d'isostéaryle, l'isononoate d'isononyle, l'isononoate d'isotridécyle, le malate de di-isostéaryle, le tétrahydroxystéarate/tétraisostéarate de dipentaérythritol, le 2-octyldodécanol, et leurs mélanges.

Selon un mode de réalisation particulière, l'huile non volatile présente dans la première composition est un mélange de polyisobutène hydrogéné, d'heptanoate d'isostéaryle, d'isononoate d'isononyle, d'isononoate d'isotridécyle, de malate de di-isostéaryle, de tétrahydroxystéarate/tétraisostéarate de dipentaérythritol et de 2-octyldodécanol.

Les huiles non volatiles peuvent être présentes dans la première ou deuxième composition en une teneur allant de 20 à 99 % en poids, notamment de 30 % à 80 % en poids, et en particulier de 40 % à 80 % en poids, par rapport au poids total de la composition.

Selon un mode particulier de réalisation, la première ou la deuxième composition comprend au moins une huile de silicone, présente en une teneur allant de 0 à 90 % en poids, notamment de 0,1 à 80 % en poids, et en particulier de 2 à 80 % en poids, par rapport au poids total de la composition.

Selon un autre mode de réalisation, l'huile de silicone est présente dans la première composition, selon un rapport pondéral, par rapport au polymère de silicone de formule générale (I), variant de 80:1, et en particulier de 60:1, et plus particulièrement de 40:1.

La première ou deuxième composition cosmétique peut également comprendre au moins un ester de saccharide et d'un acide carboxylique, comme :

- les esters d'acides gras en C8 à C22 et de saccharose, tels que ceux décrits dans le brevet US 6 238 678 et la demande WO 00/10523 dont les contenus sont incorporés dans la présente demande par référence,
- les esters d'acides en C1 à C6 et de saccharose, comme le saccharose d'acétate et d'isobutyrate commercialisé

sous la référence SAIB par Eastman SAIB.

La phase grasse liquide de la première ou de la deuxième composition peut, le cas échéant, être épaissie, gélifiée ou structurée en y incorporant un agent gélifiant de phase grasse, notamment un gélifiant décrit dans la demande publiée WO 2004/28486 dont le contenu est inclus dans la présente demande par référence.

La première ou la deuxième composition selon l'invention peut comprendre au moins un milieu aqueux, constituant une phase aqueuse, qui peut former la phase continue de la composition.

La phase aqueuse peut être constituée essentiellement d'eau.

Elle peut également comprendre un mélange d'eau et de solvant organique miscible à l'eau (miscibilité dans l'eau supérieure à 50 % en poids à 25°C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, et les aldéhydes en $C_2$-$C_4$.

La phase aqueuse (eau et éventuellement le solvant organique miscible à l'eau) peut être présente à une teneur allant de 0,1 à 40% en poids, notamment allant de 0,1 à 20 % en poids, et en particulier 0,1 à 10 % en poids, par rapport au poids total de la composition.

Cette phase aqueuse peut, le cas échéant, être épaissie, gélifiée ou structurée en y incorporant en outre un agent gélifiant aqueux traditionnel notamment d'origine minérale comme l'argile, par exemple, et/ou organique comme un polymère gélifiant aqueux.

Lorsque la première ou la deuxième composition est destinée à être appliquée sur les ongles, la composition contient de préférence un solvant choisi parmi:

- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la $\gamma$-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- les composés hétérocycliques tels que le tétrahydrofuranne ;
- le carbonate de propylène, le 3-éthoxypropionate d'éthyle ;
- leurs mélanges.

On préfère en particulier l'acétate de méthyle, l'acétate d'isopropyle, l'acétate de méthoxypropyle, le lactate de butyle, l'acétone, la méthyléthylcétone, le diacétone alcool, la $\gamma$-butyrolactone, le tétrahydrofuranne, le carbonate de propylène, le 3-éthoxypropionate d'éthyle, le diméthylsulfoxide, et leurs mélanges.

Selon une variante, les premières et deuxième compositions cosmétiques conformes à la présente invention peuvent être présentées sous la forme d'une émulsion, dans laquelle le polymère de silicone de formule générale (I), telle que définie précédemment, peut avoir la fonction de tensioactif.

Au sens de la présente invention, on entend par « émulsion », un système de deux liquides non miscibles dont l'un est finement divisé en gouttelettes dans l'autre. La phase dispersée est encore appelée « phase interne ou discontinue ». La phase dispersante est aussi appelée « phase externe ou continue ».

Les émulsions dans lesquelles la phase dispersée est lipophile, l'huile végétale ou minérale par exemple, et la phase dispersante hydrophile, par exemple l'eau, sont dites de type aqueux (H/E : huile-dans-eau). Les émulsions dans lesquelles la phase dispersée est hydrophile et la phase dispersante est lipophile sont dites de type huileux (E/H : eau-dans-huile).

Il existe également des émulsions dites multiples, par exemple E/H/E : eau-dans-huile-dans-eau.

Au sens de la présente invention, les émulsions contiennent une phase lipophile et une phase hydrophile, cette dernière n'étant pas systématiquement de l'eau.

Ainsi, la première ou la deuxième composition peuvent être sous forme d'émulsion anhydre.

En particulier, la première ou la deuxième composition peut posséder, par exemple, une phase grasse continue, pouvant contenir moins de 10 % en poids d'eau, notamment moins de 5 % en poids d'eau, voire moins de 1 % en poids d'eau par rapport au poids total de la composition.

Les première et deuxième compositions sont avantageusement anhydres, c'est-à-dire pouvant contenir moins de 5 % en particulier moins de 3 %, en particulier moins de 2 %, et plus particulièrement moins de 1 % d'eau par rapport au poids total de la composition. Elles peuvent alors se présenter notamment sous forme de gels huileux, de liquides huileux, de pâtes ou de sticks ou encore sous forme de dispersion vésiculaire contenant des liquides ioniques et/ou non ioniques.

La première et/ou la deuxième composition peuvent également comprendre au moins une cire ou un corps gras pâteux. Ces corps gras peuvent être d'origine animale, végétale, minérale ou synthétique.

Par corps gras pâteux, on entend un composé lipophile comportant à la température de 23°C une fraction liquide et une fraction solide. Par corps gras pâteux, on entend également le polylaurate de vinyle.

Par cire au sens de la présente invention, on entend un composé lipophile, solide à température ambiante (25°C), à changement d'état solide/liquide réversible, ayant un point de fusion supérieur ou égal à 30°C pouvant aller jusqu'à 120°C.

Le point de fusion de la cire peut être mesuré à l'aide d'un calorimètre à balayage différentiel (D.S.C.), par exemple le calorimètre vendu sous la dénomination DSC 30 par la Société METLER.

Les cires peuvent être hydrocarbonées, fluorées et/ou siliconées et être d'origine végétale, minérale, animale et/ou synthétique. En particulier, les cires présentent une température de fusion supérieure à 25°C et mieux supérieure à 45°C.

Comme cire utilisable dans la première ou deuxième composition, on peut citer des cires hydrocarbonées linéaires. Leur point de fusion est avantageusement supérieur à 35°C, par exemple supérieur à 55°C, de préférence supérieur à 80°C.

Les cires hydrocarbonées linéaires sont avantageusement choisies parmi les alcanes linéaires substitués, les alcanes linéaires n on substitués, les alcènes linéaires n on substitués, les alcènes linéaires substitués, un composé non substitué étant uniquement composé de carbone et d'hydrogène. Les substituants mentionnés précédemment ne contenant pas d'atomes de carbone.

Les cires hydrocarbonées linéaires incluent les polymères et copolymères de l'éthylène, de poids moléculaire compris entre 400 et 800, par exemple la Polywax 500 ou Polywax 400 commercialisée par New Phase Technologies.

Les cires hydrocarbonées linéaires incluent les cires de paraffine linéaires, comme les paraffines S&P 206, S&P 173 et S&P 434 de Strahl & Pitsch.

Les cires hydrocarbonées linéaires incluent les alcools linéaires à longue chaîne, comme les produits comprenant un mélange de polyéthylène et d'alcools en comprenant 20 à 50 atomes de carbones, notamment le Performacol 425 ou le Performacol 550 (mélange dans les proportions 20/80) commercialisés par New Phase Technologies.

Des exemples de cires siliconées sont par exemple

- Les C20-24 alkyl méthicone, C24-28 alkyl diméthicone, C20-24 alkyl diméthicone, C24-28 alkyl diméthicone commercialisées par Archimica Fine Chemicals sous la référence SilCare 41 M40, SilCare 41 M50, SilCare 41 M70 and SilCare 41M80,
- Les stéaryl diméthicone de référence SilCare 41 M65 commercialisées par Archimica ou de référence DC-2503 commercialisée par Dow-Corning
- Les stéaroxytriméthylsilane vendues sous la référence SilCare 1 M71 ou DC-580
- Les produits ABIL Wax 9810, 9800, or 2440 de Wacker-Chemie GmbH,
- Les C30-45 alkyl méthicone commercialisées par Dow Corning sous la référence AMS-C30 Wax, de même que les C30-45 alkyl diméthicone commercialisées sous la référence SF1642 ou SF-1632 par General Electric.

La nature et la quantité de ces corps gras sont fonction des propriétés mécaniques et des textures recherchées. A titre indicatif, la première ou la deuxième composition peut contenir de 0,1 à 50 % en poids de cires, par rapport au poids total de la composition et mieux de 1 à 30 % en poids.

La première et la deuxième composition peuvent comprendre en outre un polymère siliconé différent du polymère de silicone décrit précédemment, de masse moléculaire en poids est supérieure ou égale à 200 000 g/mol, de préférence compris entre 200 000 et 2 500 000, de préférence entre 200 000 et 2 000 000 g/mol.

La viscosité de ce polymère siliconé peut être comprise entre 100 000 et 5 000 000 cSt, de préférence entre 100 000 et 1 000 000 cSt, de préférence encore entre 300 000 et 700 000 cSt, mesurée selon la norme ASTM D-445.

Le polymère siliconé est de préférence choisi parmi les diméthiconols, les fluorosilicones, les diméthicones et leurs mélanges.

Le polymère siliconé de haut poids moléculaire peut être la diméthiconol commercialisée par Dow Corning dans une polydiméthylsiloxane (5 cSt) sous la référence D2-9085, la viscosité du mélange étant égale à 1 550 cSt, ou la diméthiconol

commercialisée par Dow Corning dans l'octaméthylcyclotétrasiloxane sous la référence DC 1503. On préfère également la diméthiconol (de poids moléculaire égal à 1 770 000) commercialisée par Dow Corning sous la référence Q2-1403 ou Q2-1401, la viscosité du mélange étant égale à 4 000 cSt.

Les diméthicones de haut poids moléculaire selon l'invention incluent les diméthicones décrites dans le brevet US 4 152 416. Elles sont par exemple commercialisées sous les références SE30, SE33, SE 54 et SE 76.

La première et/ou la deuxième composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la première ou deuxième composition, en une teneur allant de 0,01 % à 50 % en poids, par rapport au poids de la composition, de préférence de 0,01 % à 30 % en poids.

La première composition peut contenir un agent de coloration choisi parmi les colorants liposolubles, les colorants hydrosolubles, les pigments, les nacres et leurs mélanges.

Par « pigments », il faut comprendre des particules blanches ou colorées, minérales ou organiques, insolubles dans la phase organique liquide, destinées à colorer et/ou opacifier la composition.

Par « nacres », il faut comprendre des particules irisées, notamment produites par certains mollusques dans leur coquille ou bien synthétisées, insolubles dans le milieu de la composition.

Par « colorants », il faut comprendre des composés généralement organiques solubles dans les corps gras comme les huiles ou dans une phase hydroalcoolique.

La première et/ou la deuxième composition peut contenir un agent dispersant des pigments.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en $C_8$ à $C_{20}$ et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans la première ou deuxième composition, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

L'acide polydihydroxystéarique et les esters de l'acide hydroxy12-stéarique sont de préférence destinés à un milieu hydrocarboné ou fluoré, alors que les mélanges de diméthylsiloxane oxyéthylène/oxypropyléné sont de préférence destinés à un milieu siliconé.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi le mica recouvert de titane ou d'oxychlorure de bismuth, le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth. On peut également utiliser les pigments interférentiels, notamment à cristaux liquides ou multicouches.

Les colorants hydrosolubles sont par exemple le jus de betterave, le bleu de méthylène.

De manière avantageuse, les première et/ou deuxième compositions cosmétiques conformes à l'invention peuvent également comprendre des charges, de nature organique ou minérale, permettant notamment de leur conférer une stabilité améliorée au regard de l'exsudation.

Par « charge », il faut comprendre les particules incolores ou blanches, solides de toutes formes, qui se présentent sous une forme insoluble et dispersée dans le milieu de la composition, minérales ou organiques, destinées à

donner du corps ou de la rigidité à la composition, et/ou de la douceur, de la matité et de l'uniformité au maquillage.

Les charges utilisées dans les première ou deuxième composition peuvent être de formes lamellaires, globulaires, sphériques, de fibres ou de toute autre forme intermédiaire entre ces formes définies.

Les charges selon l'invention peuvent être ou non enrobées superficiellement, en particulier traitées en surface par des silicones, des acides aminés, des dérivés fluorés ou toute autre substance favorisant la dispersion et la compatibilité de la charge dans la composition.

Au sens de la présente invention, les termes « charges minérales » et « charges inorganiques » sont utilisés de manière interchangeable.

- Parmi les charges utilisables, on peut citer le talc, le mica, la silice, le siloxysilicate de triméthyle, le kaolin, la bentone, le carbonate de calcium précipité, le carbonate et l'hydrogéno-carbonate de magnésium, l'hydroxyapatite, le nitrure de bore, les microsphères de silice creuses (Silice Beads de Maprecos), les microcapsules de verre ou de céramique, les charges à base de silice comme l'Aerosil 200, l'Aerosil 300 ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass

et leurs mélanges.

Parmi les charges, on peut encore citer les poudres de polyamide (Nylon® Orgasol de chez Atochem), de poly-b-alanine et polyéthylène, les poudres de polytétrafluoroéthylène (Téflon®), la lauroy-lysine, l'amidon, les poudres de polymères de tétrafluoroéthylène, les microsphères creuses de polymères telles l'EXPANCEL (NOBEL INDUSTRIE), le carbonate de calcium précipité, le carbonate et l'hydrogéno-carbonate de magnésium, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, le Polypore® L 200 (Chemdal Corporation)

- les microbilles de résine de silicone (Tospearl® de Toshiba, par exemple),
- les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400® ou PLASTIC POWDER D-800® de la société TOSHIKI.

et leurs mélanges.

Les charges peuvent être présentes à raison de 0,001 à 35 % du poids total de la composition, de préférence 0,5 à 15 %.

La charge peut être par exemple une charge dont la granulométrie moyenne est inférieure à 100 $\mu$m, notamment comprise entre 1 et 50 $\mu$m, par exemple entre 4 et 20 $\mu$m.

Selon un mode de réalisation particulier, la première ou le deuxième composition selon l'invention comprend au moins une charge qui présente à raison de 0,01 % à 60 % du poids total de la composition, en particulier de 0,5 à 20 %, plus particulièrement de 1 à 10 % en poids par rapport au poids total de la composition.

La première et/ou la deuxième composition de l'invention peut, en outre, contenir un ou plusieurs actifs cosmétiques ou dermatologiques tels que ceux classiquement utilisés.

Comme actifs cosmétiques, dermatologiques, hygiéniques ou pharmaceutiques, utilisables dans la composition de l'invention, on peut citer les hydratants, vitamines telles que vitamine B3 et E, acides g ras essentiels, sphingolipides, filtres solaires. Ces actifs sont utilisés en quantité habituelle pour l'homme de l'art et notamment à des concentrations de 0 à 20 % et notamment de 0,001 à 15 % du poids total de la première ou deuxième composition.

Les compositions peuvent comprendre, en outre, tout autre additif usuellement utilisé dans de telles compositions, tel que de l'eau, des agents tensioactifs tels que décrits dans la demande FR2834452 publiée le 11 juillet 2003, dont le contenu est incorporé dans la présente demande par référence, des antioxydants, des parfums, des conservateurs et des huiles essentielles.

Bien entendu l'homme du métier veillera à choisir ce ou ces éventuels composés complémentaires, et/ou leur quantité, de manière telle que les propriétés avantageuses de la composition ne soient pas, ou substantiellement pas, altérées par l'adjonction envisagée.

Les compositions du produit peuvent se présenter sous forme d'un produit coulé et par exemple sous la forme d'un stick ou bâton, ou sous la forme de coupelle utilisable par contact direct ou à l'éponge. En particulier, elles trouvent une application en tant que fond de teint coulé, fard à joues ou à paupières coulé, rouge à lèvres, base ou baume de soin pour les lèvres, produit anti-cernes, vernis à ongles. Elles peuvent aussi se présenter sous forme d'une pâte souple ou encore de gel, de crème plus ou moins fluide, ou de liquide conditionné dans un tube.

La première et/ou la seconde composition peut être sous forme d'un liquide anhydre, d'un stick anhydre ou d'une émulsion. Les compositions du produit selon l'invention peuvent constituer notamment une composition cosmétique de soin pour le visage, pour le cou, pour les mains ou pour le corps (par exemple crème de soin, huile solaire, gel corporel), une composition de maquillage (par exemple gel de maquillage, crème, stick) ou une composition de bronzage artificiel ou de protection de la peau.

Les compositions du produit selon l'invention peuvent se présenter sous la forme d'une composition de soin de la peau et/ou des phanères ou sous forme d'une composition de protection solaire, d'hygiène corporelle, notamment sous forme de déodorant. Elles se présentent alors notamment sous forme non colorée. Elles peuvent alors être utilisées comme base de soin pour la peau, les phanères ou les lèvres (baumes à lèvres, protégeant les lèvres du froid et/ou du soleil et/ou du vent, crème de soin pour la peau, les ongles ou les cheveux).

Par cosmétiquement acceptable, on entend au sens de l'invention une composition d'aspect, d'odeur et de toucher agréables.

Chaque composition du produit selon l'invention peut se présenter sous toute forme galénique normalement utilisée pour une application topique et notamment sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules, les vésicules étant situés à l'interface huile/eau, ou d'une poudre. Chaque composition peut être fluide ou solide.

Selon un mode de mise oeuvre, la première ou la seconde composition, ou les deux, sont à phase grasse continue et de préférence sous forme anhydre et peuvent contenir moins de 5% d'eau, et encore mieux moins de 1% d'eau par rapport au poids total de la première ou seconde composition.

Chaque première et seconde composition peut avoir l'aspect d'une lotion, d'une crème, d'une pommade, d'une pâte souple, d'un onguent, d'un solide coulé ou moulé et notamment en stick ou en coupelle, ou encore de solide compacté.

Chaque composition peut être conditionnée séparément dans un même article de conditionnement par exemple dans un stylo bi-compartimenté, la composition de base étant délivrée par une extrémité du stylo et la composition du dessus étant délivrée par l'autre extrémité du stylo, chaque extrémité étant fermée notamment de façon étanche par un capuchon.

Alternativement, chacune des compositions peut être conditionnée dans un article de conditionnement différent.

De préférence, la composition qui est appliquée en première couche est sous forme liquide ou pâteuse, ce qui est hautement souhaitable dans le cas d'un rouge à lèvres ou d'un eyeliner.

Le produit selon l'invention peut être avantageusement utilisé pour le maquillage de la peau et/ou des lèvres et/ou des phanères selon la nature des ingrédients utilisés. En particulier, le produit de l'invention peut se présenter sous forme de fond de teint solide, de bâton ou pâte de rouge à lèvres, de produit anti-cernes, ou contours des yeux, d'eye liner, de mascara, d'ombre à paupières, de produit de maquillage du corps ou encore un produit de coloration de la peau.

Avantageusement, la deuxième composition présente des propriétés de soin, de brillance ou de transparence.

L'invention a encore pour objet un produit à lèvres, un vernis, un mascara, un fond de teint, un tatouage, un fard à joues ou à paupières comprenant une première et une seconde compositions telles que décrites précédemment.

Les compositions du produit de l'invention peuvent être obtenues par chauffage des différents constituants à la température de fusion des cires les plus élevées, puis coulage du mélange fondu dans un moule (coupelle ou doigt de gant). Elles peuvent aussi être obtenues par extrusion comme décrit dans la demande EP-A-0 667 146.

L'invention est illustrée plus en détail dans les exemples suivants. Les pourcentages sont des pourcentages en poids.

**Exemple 1 :**

Première composition : Rouge à lèvres

| | |
|---|---|
| Cire de polyéthylène (Masse moléculaire en poids 500) | 10,5 % |
| Alcools gras linéaires (PERFORMACOL 550 ALCOHOL commercialisé par New Phases Technologies) | 2,5 % |
| Dispersion de copolymère acrylate de méthyle/acide acrylique | 68 % |
| Acetate isobutyrate de saccharose (EASTMAN SAIB commercialisé par EASTMAN CHEMICAL) | 5 % |
| Pâte pigmentaire | 13,5 % |
| Parfum | 0,5 % |

*Préparation de la dispersion de copolymère*

On prépare une dispersion de copolymère non réticulé d'acrylate de méthyle et d'acide acrylique dans un rapport 95/5, dans l'isododécane, selon la méthode de l'exemple 1 du document EP-A-749 746, en remplaçant l'heptane par l'isododécane. On obtient ainsi une dispersion de particules de poly(acrylate de méthyle/acide acrylique) stabilisées en surface dans l'isododécane par un copolymère dibloc séquencé polystyrène/copoly(éhylène-propylène) vendu sous le nom de Kraton G1701, ayant un taux de matière sèche de 25% en poids.

*Préparation du rouge à lèvres*

Dans un poêlon on introduit la cire de polyéthylène, les alcools en C30-50 et la pâte pigmentaire que l'on chauffe à 100°C sous agitation magnétique afin d'obtenir un mélange homogène. La pâte pigmentaire est constituée de 70 % de pigments, 1 % de stéarate d'acide poly(12-hydroxystéarique) et 29 % de polyisobutène hydrogéné.
On ajoute ensuite l'acétate isobutyrate de saccharose et la dispersion selon l'exemple 1 et tout en maintenant la température et l'agitation. La composition est coulée dans des moules à 40°C qui sont placés à -20°C pendant trente minutes. On procède ensuite au démoulage des sticks. Les sticks obtenus sont homogènes en teinte et glissant à l'application. Ils conduisent à un dépôt sur les lèvres de bonne tenue, ne migrant pas, non transfert, ne collant pas.

<u>Deuxième composition : Baume pour les lèvres</u>

| | Pourcentages en poids |
|---|---|
| Phényltriméthicone (DC 556 deDow Corning) | 25 |
| Polydiméthylsiloxane (Belsil 1000) | 25% |
| Diméthicone de polyglycéryl-3 polydiméthylsiloxyéthyle (KF6104 ® de Shin Etsu) | 12% |
| Polydiméthylsiloxane (DC 200, 10 cSt) | 15% |
| Octyldodécylnéopentanoate | 15% |
| Silice hydophobe (Aerosil R 972) | 8% |

**<u>Exemple 2</u>** :

<u>Première composition : Rouge à lèvres</u>

On prépare un rouge à lèvres comprenant la diméthicone de polyglycéryl-3 polydiméthylsiloxyéthyle commercialisée sous la référence KF6104 par SHIN-ETSU, et dont la composition est précisée en tableau I.

Tableau I

| | Pourcentages massiques |
|---|---|
| Polyisobutène hydrogéné (Parleam HV de NOF) | 4 |
| Isononanoate d'isononyle | 12 |
| 2-Octyldodécanol | 4,5 |
| Malate de diisostéaryle | 33,9 |
| Triglycérides d'acides lauriques/palmitique/cétylique/stéarique 50/20/10/10 (Softisan 100 ®de Sasol) | 4 |
| N-lauroyl L-lysine | 1 |
| Copolymère d'acrylates/acrylate de stéaryle/méthacrylate de diméthicone (KP 561 P ® de Shin Etsu) | 4 |
| Diméthicone 6 cSt (KF 96) | 4 |
| Diméthicone de polyglycéryl-3 polydiméthylsiloxyéthyle (KF6104 ® de Shin Etsu) | 13 |
| Conservateur | qs |
| Cire de Polyéthylène (PM 500) | 6,6 |
| Cire de polyéthylène (PM 400) | 3,8 |
| Silice pyrogénée hydrophobe traitée en surface par di-méthylsilane (Aerosil R 972 ® de Degussa) | 2 |
| Pigments | 7,0 |
| Siméthicone (Antifoam C ®de Dow Corning) | 0,2 |
| Total | 100 |

*Mode opératoire*

Une phase huileuse est préparée en mélangeant à chaud (environ 95 °C) l'isononanoate d'isononyle, le 2-octyldodécanol, le malate de diisostéaryle, avec la diméthicone de polyglycéryl-3 polydiméthylsiloxyéthyle et l'huile dimethicone. La phase huileuse ainsi préparée est maintenue sous agitation à environ 95 °C, et les charges (N-lauroyl L-lysine et silice pyrogénée) sont ajoutées au mélange.

Puis sont rajoutés au mélange les cires, les pigments sous la forme d'une pâte pigmentaire, le polyisobutène hydrogénée et la siméthicone.

Deuxième composition : Stick incolore pour les lèvres

| | % en poids |
|---|---|
| Huile de silicone (PDMS) DC200 de Dow Corning (5 cSt) | 25 |
| DIMETHICONE (and) DIMETHICONOL D2-9085 de Dow Corning (1550 cSt) | 61 |
| TRIFLUOROPROPYL DIMETHICONE (100 cSt) X22-819 de Shin Etsu | 1 |
| C30-45 ALKYL DIMETHICONE (SF 1642 de GE Bayer Silicone) | 5 |
| Cire de POLYETHYLENE (PM en poids 500) | 8 |

L'huile de silicone, la diméthiconol et la diméthicone fluorée sont mélangées à chaud jusqu'à former un mélange homogène. On ajoute ensuite la $C_{30}$-$C_{45}$ alkyl diméthicone dans le mélange précédent porté à 110°C. La cire de polyéthylène est ensuite ajoutée peu à peu jusqu'à obtention d'un mélange homogène. Le mélange est refroidi à 90-95°C puis versé dans les moules, qui sont placés à -20°C pendant trente minutes. On procède enfin au démoulage des sticks.

**Exemple 3 :**

Première composition : Stick de rouge à lèvres

| Ingrédients | % massique |
|---|---|
| Pigments | 8,20 |
| Hydrogenated polyisobutene (Parleam) | 5,18 |
| Polyhydroxystearic acid (Octacare DSPOL300, Avecia) | 0,21 |
| C30-C50 alcohols (Performacol 550 Alcohol, New Phase Tecnologies) | 2 |
| Polyethylene (Polywax 500, Bareco) | 10 |
| Sucrose acetate isobutyrate (Eastman SAIB Special, Eastman Chemical) | 5 |
| Polymère d'acrylate de méthyle greffé | 68,82 |
| parfum | qs |

*Préparation du polymère obtenu par polymérisation d'acrylate de méthyle et du macromonomère correspondant à un copolymère polyéthylène/polybutylène à groupement terminal méthacrylate (Kraton L-1253).*

Dans un réacteur, on charge 2 kg d'heptane, 2 kg d'isododécane, 2,8 kg d'acrylate de méthyle et 1,2 kg de macromonomère du type copolymère de polyéthylène/polybutylène à groupement terminal méthacrylate (Kraton L-1253) et 320 g de tertio butyl peroxy-2-éthylhexanoate (Trigonox 21 S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 16 kg d'acrylate de méthyle et 200 g de Trigonox 21S.

On laisse ensuite le chauffage pendant 4 heures à 90°C puis on distille l'heptane du milieu réactionnel. A l'issue de cette opération de distillation, on obtient une dispersion de particules de polymère ainsi préparé stable dans l'isododécane

Le polymère greffé comprend 6% en poids de macromonomère par rapport au poids du polymère.

Les caractéristiques du polymère et des particules formées par ledit polymère sont les suivantes :

- Masse moléculaire poids Mw = 119900
- Masse moléculaire nombre Mn = 16300
- Indice de polydispersité (Mw/Mn) = 7.37
- Transition vitreuse : 10°C par DSC Mettler ;
- Extrait sec : 52.4 % dans l'isododécane, réalisé par thermobalance ;
- Granulométrie : 46 nm avec polydispersité de 0,05 réalisée sur Malvern Autosizer Lo-C à 25°C

*Préparation du rouge à lèvres*

Dans un poêlon, sous agitation Rayneri, on introduit les cires, les pâtes pigmentaires et l'ester de saccharose, on se place à 105°C, on laisse tourner 30 minutes, puis on ajoute les nacres, on ajoute ensuite la dispersion de polymère et le parfum, on laisse tourner 10 minutes, puis on coule dans un moule à 42°C. On place le moule au congélateur et on démoule lorsque le moule est à environ 4°C.

Deuxième composition : Baume pour les lèvres

| | Pourcentages en poids |
|---|---|
| Cire de polyéthylène | 2 |
| Cire d'ozokérite | 3 |
| Cire d'abeilles | 3 |

Suite de tableau

| | Pourcentages en poids |
|---|---|
| Cire microcristalline | 2 |
| Cire de paraffine (EMW-3 de la société Nippon Seiro) | 8 |
| Sucrose polycottonseedate | 5 |
| Polyisobutene hydrogéné | 10 |
| Diisostéaryl malate | 10 |
| Neopentyl glycol dicaprate | 10 |
| Isotridecyl isononanoate | 10 |
| Copolymère d'acrylates/acrylate de stéaryle/méthacrylate de diméthicone (KP 561 P ® de Shin Etsu) | 2 |
| Diméthicone de polyglycéryl-3 polydiméthylsiloxyéthyle (KF6104® de Shin Etsu) | 10 |
| Trimethyl siloxy silicate (SR 1000®) | 2 |
| Dimethicone 6 cSt | 5 |
| Mica recouvert de titane | 5 |
| Pigment réfléchissant (Metashine ® de Nippon Sheet Glass) | 2 |
| Dioxyde de titane | 1 |

## Revendications

**1.** Produit cosmétique comprenant une première et une seconde compositions, la première composition contenant au moins un polymère de silicone de formule générale (I) :

$$R^1_a\ R^2_b\ R^3_c\ SiO_{(4-a-b-c)/2} \qquad (I)$$

dans laquelle :

- a, b et c sont tels que a varie de 1 à 2,5, b et c, indépendamment l'un de l'autre, varient de 0,001 à 1,5,
- $R^1$ identique ou différent, est choisi parmi :

  - les radicaux alkyle en $C_1$-$C_{30}$, le cas échéant substitué par un ou plusieurs atomes de fluor, groupements amino et/ou carboxy, aryle, aralkyle, et
  - les radicaux de formule générale (II) :

  $$-C_dH_{2d}-O-(C_2H_4O)_e(C_3H_6O)_fR^4 \qquad (II)$$

  avec :

    - $R^4$ est un radical hydrocarboné en $C_1$ à $C_{30}$, ou un radical $R^5$-(CO)- avec $R^5$ étant un radical hydrocarboné en $C_1$ à $C_{30}$, et
    - d, e et f sont des entiers tels que d varie de 0 à 15, e et f, indépendamment l'un de l'autre, varient de 0 à 50,

  - et leurs combinaisons,

- $R^2$ est un radical représenté par la formule générale (III) :

$$-Q-O-X \qquad (III)$$

avec :

  - Q étant un radical hydrocarboné bivalent en $C_2$-$C_{20}$ pouvant inclure au moins une liaison éther et/ou au moins une liaison ester et

- X étant un radical hydrocarboné polyhydroxylé,

- $R^3$ est radical choisi parmi

- les radicaux alkyle en $C_1$ à $C_{30}$, le cas échéant substitués par un ou plusieurs atomes de fluor, groupements amino et/ou carboxy,
- les radicaux aryle, aralkyle,
- les groupements organosiloxane de formule générale (IV) :

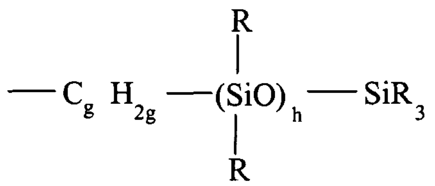

(IV)

avec :
- chacun des radicaux R représentant un radical alkyle en $C_1$-$C_{30}$, le cas échéant substitué par un ou plusieurs atomes de fluor, les radicaux aryle, aralkyle,
- g et h étant des entiers tels que g varie de 1 à 5 et h varie de 0 à 500,

et la seconde composition distincte de la première comprenant un milieu cosmétiquement acceptable.

**2.** Produit selon la revendication précédente, **caractérisé en ce que** le polymère de silicone est un composé de formule générale (I) dans laquelle $R_1$ est un radical alkyle en $C_1$ à $C_{10}$, en particulier de $C_1$ à $C_6$, et en particulier en $C_1$ à $C_4$.

**3.** Produit selon la revendication 1 ou 2, **caractérisé en ce que** le polymère de silicone est un composé de formule générale (I) dans laquelle :

- a varie de 1 à 1,4, b et c, indépendamment l'un de l'autre, varient de 0,02 à 0,04, et
- $R^1$ est un radical alkyle en $C_1$ à $C_{10}$, en particulier de $C_1$ à $C_6$, et en particulier en $C_1$ à $C_4$,
- $R^2$ est représenté par la formule (IIIA) :

$$-C_3H_6O[CH_2CH(OH)CH_2O]_nH \qquad \text{(IIIA)}$$

dans laquelle n varie de 1 à 5,
- $R^3$ est représenté par la formule (IVA) :

$$-C_2H_4(CH_3)_2SiO[(CH_3)_2SiO]_mSi(CH_3)_2 \qquad \text{(IVA)}$$

dans laquelle m varie de 3 à 9.

**4.** Produit selon la revendication 3, **caractérisée en ce que** le polymère de silicone est un composé de formule générale (I) :

$$R^1_a\, R^2_b\, R^3_c\, SiO_{(4\text{-}a\text{-}b\text{-}c)/2} \qquad \text{(I)}$$

dans laquelle :

- a varie de 1 à 1,4, b et c, indépendamment l'un de l'autre, varient de 0,02 à 0,04, et
- $R^1$ est un radical méthyle,
- $R^2$ est représenté par la formule (IIIA) dans laquelle n varie de 1 à 5, et
- $R^3$ est représenté par la formule (IVA) dans laquelle m varie de 3 à 9.

**5.** Produit selon l'une des revendications précédentes, **caractérisé en ce que** -Q- est un radical bivalent choisi parmi

$-(CH_2)_2-$ et $-(CH_2)_3-$..

6. Produit selon l'une des revendications précédentes, **caractérisé en ce que** X est un radical hydrocarboné polyhydroxylé comprenant au moins deux résidus hydroxyles, et en particulier un groupement hydrocarboné choisi parmi les dérivés glycérylés et les dérivés osidiques.

7. Produit selon la revendication précédente, **caractérisé en ce que** Q-O-X est un radical correspondant à l'une des formules suivantes :

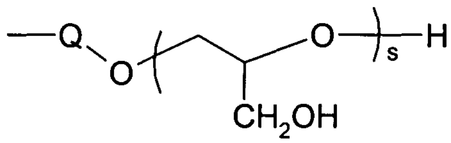

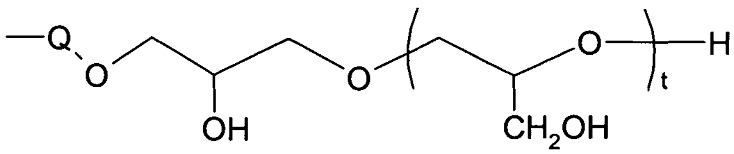

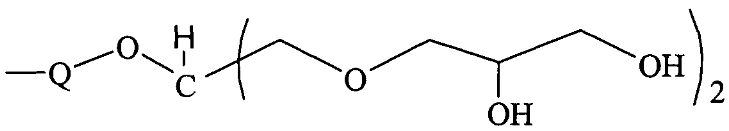

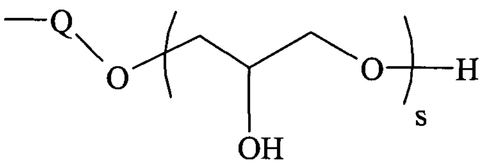

dans lesquelles Q a la même signification que dans la formule générale (III) selon l'une des revendications précédentes, et s et t sont des entiers compris dans l'intervalle variant de 1 à 20, en particulier de 1 à 15, en particulier de 1 à 10, et plus particulièrement de 1 à 5.

8. Produit selon l'une des revendications précédentes, caratérisée en ce que le polymère de silicone est une diméthicone de polyglycéryl-3 polyméthylsiloxyéthyle de formule (V) :

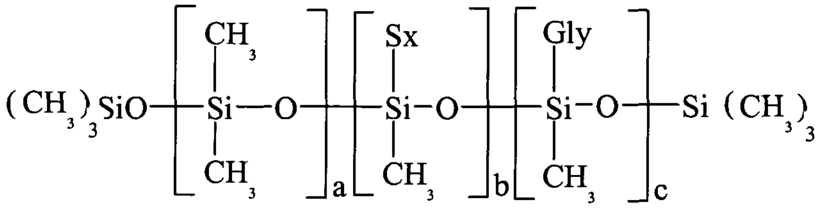 (V)

dans laquelle :

Sx : $-C_2H_4\ [(CH_3)_2SiO]_mSi(CH_3)_3$
Gly : $-C_3H_6O[CH_2-CH(OH)CH_2O]_nH$
avec a = 1-1,4, b = 0,02-0,04, c = 0,02-0,04, m = 3-9, n = 1-5

9. Produit selon l'une des revendications 1 à 8, caratérisée en ce que le polymère de silicone est une diméthicone de

lauryl polyglycéryl-3 polyméthylsiloxyéthyle de formule (VI) :

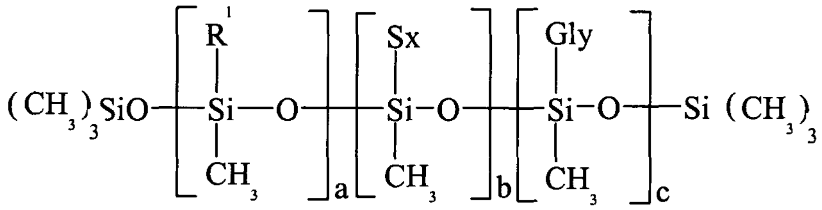

(VI)

dans laquelle Sx, Gly, a, b, c, m et n ont la même signification qu'à la revendication 7 et $R_1$ est, soit un radical méthyle, soit un radical lauryle.

**10.** Produit selon l'une des revendications 1 à 8, caratérisée en ce que le polymère de silicone est une diméthicone de polyglycéryl-3 disiloxane (formule (VII)) :

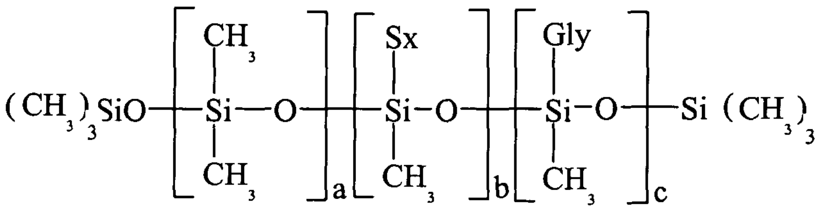

(VII)

dans laquelle Gly, a, b, c, m et n ont la même signification que précédemment, et Sx : $-O(CH_3)_2SiO-Si(CH_3)_3$

**11.** Produit selon l'une des revendications précédentes, **caractérisée en ce que** le polymère de silicone de formule générale (I) est présent dans la première composition à raison de 0,1 à 40 % en poids, en particulier de 0,5 à 30 % en poids, plus particulièrement de 1 à 25 % en poids, en particulier de 5 à 20 % en poids, en particulier de 7 à 15 % en poids, par rapport au poids total de la composition.

**12.** Produit selon l'une des revendications précédentes, **caractérisée en ce que** le polymère de silicone est tel, que lorsqu'il est présent en quantité suffisante dans la première composition, le produit est apte à former un dépôt ayant une valeur de transfert ΔE (T) au moins inférieure ou égale à 45, en particulier inférieure ou égale à 40, notamment inférieure ou égale à 35, notamment comprise entre 0 et 45.

**13.** Produit selon l'une des revendications précédentes, **caractérisée en ce que** le polymère de silicone est tel, que lorsqu'il est présent en quantité suffisante dans ladite composition, cette dernière est apte à former un dépôt ayant une valeur de tenue de la couleur à l'eau ΔE (E) au moins inférieure ou égale à 15, en particulier au moins inférieure ou égale à 10, et plus particulièrement au moins inférieure ou égale à 6.

**14.** Produit selon l'une des revendications précédentes, **caractérisée en ce que** le polymère de silicone est tel, que lorsqu'il est présent en quantité suffisante dans ladite composition, cette dernière est apte à former un dépôt ayant une valeur de tenue de la couleur à l'huile au moins inférieure ou égale à 25, au moins inférieure ou égale à 10, au moins inférieure ou égale à 8.

**15.** Produit selon l'une des revendications précédentes, **caractérisée en ce que** le polymère de silicone est tel, que lorsqu'il est présent en quantité suffisante dans ladite composition, cette dernière est apte à former un dépôt ayant une brillance moyenne supérieure ou égale à 30, voire 40, ou 45.

**16.** Produit selon l'une des revendications précédentes, **caractérisée ce que** le polymère de silicone de formule générale (I) est tel, que lorsqu'il est en quantité suffisante dans la composition cosmétique l'indice de confort d'un dépôt du produit, une fois étalée sur un support, est égale ou supérieure à 80 sur 100, de préférence supérieur à 90 sur 100, de préférence supérieur à 95 sur 100.

**17.** Produit selon la revendication précédente, **caractérisée en ce que** la première et la deuxième composition comprennent indépendamment l'une de l'autre une huile siliconée non volatile choisie parmi les polydiméthylsiloxanes (PDMS) non volatiles, comme les polydiméthylsiloxanes comportant des groupements alkyle ou alcoxy pendants et/ou en bouts de chaîne siliconée, groupements ayant chacun de 2 à 24 atomes de carbone, et les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, et leurs mélanges.

**18.** Produit selon l'une des revendications précédentes, **caractérisé en ce que** la première ou la deuxième composition contiennent indépendamment l'une de l'autre une huile hydrocarbonée choisie parmi les esters hydrocarbonés comprenant moins de 40 atomes de carbone de formule RCOOR' dans laquelle RCOO représente un reste d'acide gras comportant de 2 à 28 atomes de carbone, et R' représente une chaîne hydrocarbonée contenant de 1 à 28 atomes de carbone.

**19.** Produit selon l'une des revendications précédentes, **caractérisé en ce que** la première composition contient une huile hydrocarbonée choisie parmi le polyisobutène hydrogéné, l'heptanoate d'isostéaryle, l'isononoate d'isononyle, l'isononoate d'isotridécyle, le malate de di-isostéaryle, le tétrahydroxystéarate/tétraisostéarate de dipentaérythritol, le 2-octyldodécanol, et leurs mélanges.

**20.** Produit selon l'une des revendications précédentes, **caractérisé en ce que** la première composition contient un polymère filmogène siliconé.

**21.** Produit selon la revendication 20, **caractérisé en ce que** le polymère filmogène est choisi parmi les siloxysilicates, les polysilesquioxanes et les polyméthylsesquioxanes.

**22.** Produit selon la revendication 20, **caractérisé en ce que** le polymère filmogène est choisi parmi les copolymères comportant des groupements carboxylates et des groupements polydiméthylsiloxanes.

**23.** Produit selon la revendication 22, **caractérisé en ce que** le polymère filmogène est choisi les copolymères d'acide acrylique et d'acrylate de stéaryle à greffons polydiméthylsiloxane, les copolymères de méthacrylate de stéaryle à greffons polydiméthylsiloxane, les copolymères d'acide acrylique et de méthacrylate de stéaryle à greffons polydiméthylsiloxane, les copolymères de méthacrylate de méthyle, méthacrylate de butyle, d'acrylate d'éthyl-2-hexyle et de méthacrylate de stéaryle à greffons polydiméthylsiloxane

**24.** Produit selon l'une des revendications précédentes, **caractérisé en ce que** la première et/ou la deuxième composition contient au moins une cire et au moins un composé pâteux.

**25.** Produit selon la revendication précédente, **caractérisée en ce que** la seconde composition est transparente.

**26.** Produit selon l'une des revendications précédentes, **caractérisé en ce que** la seconde composition contient moins de 5% de pigments, de préférence moins de 2%, de préférence encore moins de 1%.

**27.** Produit selon l'une des revendications précédentes, **caractérisé en ce que** la deuxième composition est choisie de telle manière à améliorer au moins une propriété cosmétique de la première composition.

**28.** Produit selon l'une des revendications précédentes, **caractérisé en ce que** la première composition contient un agent de coloration choisi parmi les colorants liposolubles, les colorants hydrosolubles, les pigments, les nacres et leurs mélanges.

**29.** Produit selon l'une des revendications précédentes, **caractérisé en ce que** la première et/ou la seconde composition est sous forme d'un liquide anhydre, d'un stick anhydre ou d'une émulsion.

**30.** Produit selon l'une des revendications précédentes, **caractérisé en ce qu'**il se présente sous la forme d'un fond de teint, d'un fard à joues ou à paupières, d'un rouge à lèvres, d'un produit ayant des propriétés de soin, d'un mascara, d'un eye-liner, d'un vernis à ongles, d'un produit anti-cernes, ou d'un produit de maquillage du corps.

**31.** Procédé de maquillage de la peau et/ou des lèvres et/ou des phanères consistant à appliquer sur la peau, les lèvres et/ou les phanères une première couche d'une première composition comprenant un polymère de silicone comme défini dans l'une des revendications 1 à 1 0 et un agent de coloration, puis à appliquer, sur tout ou partie de ladite

première couche, une seconde couche d'une seconde composition comprenant un milieu cosmétiquement acceptable.

**32.** Kit de maquillage comprenant un produit selon l'une des revendications 1 à 30, chacune des première et seconde compositions étant conditionnées de manière séparée dans des premier et second compartiments.

**33.** Kit de maquillage selon la revendication 32, **caractérisé en ce que** les premier et second compartiments sont formés à l'intérieur d'un même récipient.

**34.** Kit de maquillage selon la revendication 33 **caractérisé en ce que** les premier et second compartiments sont formés dans deux récipients distincts.

**35.** Utilisation d'un produit cosmétique comprenant première et une seconde compositions, la première composition comprenant un polymère de silicone comme défini dans l'une des revendications 1 à 10, et la seconde composition comprenant un milieu cosmétiquement acceptable, l'une au moins desdites compositions contenant un agent de coloration, pour conférer à la peau et/ou les lèvres et/ou les phanères un maquillage confortable, brillant, non transfert et/ou non migrant et/ou de bonne tenue de la couleur et/ou de bonne tenue de la brillance.

Office européen des brevets

# RAPPORT DE RECHERCHE EUROPEENNE

Numéro de la demande
EP 05 29 1151

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y | EP 1 142 551 A (SHISEIDO CO) 10 octobre 2001 (2001-10-10) * le document en entier * ----- | 1-35 | A61K7/027 A61K7/032 A61K7/043 |
| Y | EP 1 416 016 A (SHIN-ETSU CHEMICAL CO) 6 mai 2004 (2004-05-06) * revendications 1-37; exemple 30 * ----- | 1-35 | |
| A | EP 1 249 223 A (L'ORÉAL) 16 octobre 2002 (2002-10-16) * revendications 1-73 * ----- | 1 | |
| | | | DOMAINES TECHNIQUES RECHERCHES (Int.Cl.7) |
| | | | A61K |

Le présent rapport a été établi pour toutes les revendications

1

| Lieu de la recherche | Date d'achèvement de la recherche | Examinateur |
|---|---|---|
| La Haye | 20 octobre 2005 | Willekens, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 05 29 1151

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-10-2005

| Document brevet cité au rapport de recherche | | Date de publication | Membre(s) de la famille de brevet(s) | | | Date de publication |
|---|---|---|---|---|---|---|
| EP 1142551 | A | 10-10-2001 | WO | 0134101 | A1 | 17-05-2001 |
| | | | JP | 2001131024 | A | 15-05-2001 |
| | | | TW | 550083 | B | 01-09-2003 |
| | | | US | 2005129649 | A1 | 16-06-2005 |
| EP 1416016 | A | 06-05-2004 | US | 2004091439 | A1 | 13-05-2004 |
| EP 1249223 | A | 16-10-2002 | BR | 0201262 | A | 11-03-2003 |
| | | | CA | 2380789 | A1 | 10-10-2002 |
| | | | CN | 1382431 | A | 04-12-2002 |
| | | | FR | 2823102 | A1 | 11-10-2002 |
| | | | JP | 2002322020 | A | 08-11-2002 |

EPO FORM P0460

Pour tout renseignement concernant cette annexe : voir Journal Officiel de l'Office européen des brevets, No.12/82